# EUROPEAN PATENT SPECIFICATION

(11) **EP 0 982 386 B1**
(45) Date of publication and mention of the grant of the patent: **16.04.2003**
(21) Application number: 98115835.5
(22) Date of filing: 21.08.1998
(51) Int. Cl.: C09K 19/04, C09K 19/30, C09K 19/34, C07C 25/18

(54) **Liquid crystal compound having negative dielectric anisotropy, liquid crystal composition containing said liquid crystal compound and liquid crystal display device using said composition**
Flüssigkristallverbindung mit negativer dielektrischer Anisotropie, Flüssigkristallzusammensetzung und Flüssigkristallanzeigevorrichtung
Composé liquide cristallin ayant une anisotropie diélectrique négative, composition liquide cristalline et dispositif d'affichage

(43) Date of publication of application: 01.03.2000
(73) Proprietor: Chisso Corporation, Osaka-shi Osaka 530-0055 (JP)
(72) Inventor: Miyazawa, Kazutoshi, Chiba-ken 276-022 (JP); Takeuchi, Hiroyuki, Ichihara-shi, Chiba-ken (JP); Yano, Hitoshi, Ichihara-shi, Chiba-ken (JP)
(74) Representative: Müller-Boré & Partner Patentanwälte

(56) References cited:
- WO-A-96/33251
- GB-A- 2 216 523
- CHEMICAL ABSTRACTS, vol. 129, Columbus, Ohio, US; abstract no. 268014, MIYAZAWA, KAZUTOSHI ET AL: "Liquid crystal compound with negative dielectric anisotropy, its composition, and liquid crystal display using the composition" XP002090165 & JP 10 237024 A (CHISSO CORP., JAPAN)

## Description

The present invention mainly relates to a novel liquid crystal compound having characteristic properties favorable for a liquid crystal composition used for liquid crystal display devices of various display modes such as vertically aligned mode, IPS (in-plane switching) mode, TFT (thin film transistor) mode, TN (twisted nematic) mode or STN (super twisted nematic) mode, in particular, vertically aligned mode and IPS mode, a liquid crystal composition using the liquid crystal compound and having suitable characteristics and a liquid crystal display device produced using the liquid crystal composition. In the present invention, the term "liquid crystal compound" is a general term for compounds capable of exhibiting liquid crystal phases and compounds which do not exhibit any liquid crystal phase, but are useful as ingredients for liquid crystal compositions.

Up to this time, there have been proposed various liquid crystal display modes. As an example thereof, there can be listed the following display modes (see "Up-To-Date Liquid Crystal Technique", edited by Kogyochosa Kai, 1983). Examples of modes which make use of liquid crystal compositions having positive dielectric anisotropy are TN modes, STN modes, or AM (active matrix) modes based on TN (such as TFT or MIM (metal-insulating film-metal) modes). Moreover, as modes which make use of liquid crystal compositions having negative dielectric anisotropy, there may be listed, for instance, ECB (Electric field-control birefringent effect) modes (such as HAN (hybrid molecular arrangement) mode or DAP (vertical molecular arrangement) mode), DS (dynamic scattering) mode, GH (guest-host) mode or PC (phase transition) mode.

Among these modes, the mainstreams thereof which have presently been used are those which make use of liquid crystal compositions having positive dielectric anisotropy. Contrary to this, there has been a delay in the practical application of the modes utilizing liquid crystal compositions having negative dielectric anisotropy. In connection with this, the development of liquid crystal compositions having negative dielectric anisotropy and compounds per se used in the compositions have also been insufficient as compared with the development of such compositions having positive dielectric anisotropy and compounds used in these compositions.

Under these circumstances, there have recently been reported wide variety of attempts, in particular, to eliminate one of disadvantages of the liquid crystal display techniques, i.e., the narrowness of the viewing angle. An example thereof is IPS mode (see, for instance, R. Kiefer et al., JAPAN DISPLAY '92, 547 (1992); M. Oh-e et al., ASIA DISPLAY '95, 577 (1995); TOKUHYO Hei 5-505247; Japanese Un-Examined Patent Publication (hereinafter referred to as "J.P. KOKAI") No. Hei 7-12864 7). One of the characteristic properties of IPS mode is that a comb-like electrode is arranged only on one substrate in the liquid crystal panel, while electrodes are formed on the both upper and lower substrates in the conventional liquid crystal panel. Another characteristic property of the mode is that a liquid crystal composition can be used irrespective of the dielectric anisotropy of the composition (positive or negative).

As another example of the attempts to extend the viewing angle, there may be listed a mode wherein the viewing angle is improved by making use of the vertical alignment of liquid crystal molecules (see, for instance, J.P. KOKAI No. Hei 2-176625). One of the characteristic properties of this mode is to use a liquid crystal composition having negative dielectric anisotropy.

With these points as background, it has intensively been required for the development of a liquid crystal compound having negative dielectric anisotropy and a liquid crystal composition.

Incidentally, in all of the display modes, the liquid crystal compositions used therein should have not only an appropriate dielectric anisotropy value, but also the best-controlled other characteristic properties such as values of optical anisotropy (Δn), elastic constant ratio: K₃₃/K₁₁ (K₃₃: bend elastic constant; K₃₃: spray elastic constant) and further they must satisfy such requirements that the temperature of the liquid crystal phase falls within an appropriate range and that they should have a low viscosity even at a low temperature.

None of conventionally known liquid crystal compounds satisfy all of these requirements when they are used alone. For this reason, a composition which can be used as a liquid crystal material has usually been prepared by mixing several kinds to twenty or so kinds of compounds capable of exhibiting liquid crystal phases and optionally several kinds of compounds incapable of exhibiting any liquid crystal phase. Therefore, each liquid crystal compound should also satisfy such requirements that it has good miscibility with other liquid crystal compounds and that it also has good miscibility therewith in a low temperature range since the composition is also used in low temperature **environments.**

As has been described above, however, it has been the mainstream to use liquid crystal compositions comprising liquid crystal compounds having positive dielectric anisotropy in the conventional display modes and the development of liquid crystal compositions and compounds having negative dielectric anisotropy have still been insufficient. Therefore, the conventional liquid crystal compounds and compositions have not been able to completely cope with such diversified modes and related various characteristics. For instance, they suffer from the following various problems:
· They have negative dielectric anisotropy, but the absolute value thereof is small;
· They do not permit the reduction of the driving potential of the display devices because of their large elastic constants;
· They cannot be used in a large amount because of poor miscibility with other ingredients for. the liquid crystal compositions and the display devices using them;
· It is difficult to freely establish the optical anisotropy of these compositions or compounds;
· They have high viscosities; and
· They are physically and chemically less stable.

An example of the conventionally known liquid crystal compounds having negative dielectric anisotropy is a liquid crystal compound having a 2,3-difluorophenylene group (J.P. KOKAI No. Sho 57-114532). However, the absolute values of the negative dielectric anisotropy of these compounds are not always sufficiently large and the compounds do not have desired characteristic properties such as a low elastic constant, miscibility with other ingredients, a degree of freedom of optical anisotropy, a low viscosity and physicochemical stability. For this reason, there has been desired for further improvement of these compounds and compositions.

In addition, there has not been reported any investigation to improve the foregoing characteristic properties of the compound having a 2,3-difluorophenylene group, for instance, any satisfactory attempt to increase the absolute value of the negative dielectric anisotropy thereof. In particular, there has not been proposed such an idea that an ester derivative skeleton of a fluoro- or chloro-carboxylic acid is introduced into the compound having a 2,3-difluorophenylene group.

As has been described above, the compounds of the present invention cannot easily be hit upon on the basis of the teachings or knowledges of the prior art.

Accordingly, an object of the present invention is to provide a novel liquid crystal compound which can be not only used in various display modes utilizing compounds or compositions having negative dielectric anisotropy, such as vertically aligned mode as disclosed in J.P. KOKAI No. Hei 2-176625, IPS, ECB (such as HAN or DAP), DS, GH or PC mode, but also used for controlling various characteristics of liquid crystal compositions for various display modes utilizing compounds or compositions having positive dielectric anisotropy, such as TN, STN, or TN based AM (TFT or MIM) mode, and which can solve the foregoing problems, as well as a liquid crystal composition containing the compound and a liquid crystal display device produced using the composition.

The inventors of this invention have conducted various studies to accomplish the foregoing objects, as a result, have found that the compounds as specified in the following first aspect and related embodiments of the present invention not only have negative dielectric anisotropy whose absolute values are large, but also have small elastic constants, good miscibility with other substances, low viscosities and high physicochemical stability, that the use of the foregoing compounds permits to prescribe the formation of liquid crystal compositions whose elastic constant is small, whose dielectric and optical anisotropy values can appropriately be established, which have low viscosities and excellent physicochemical stability and, in particular, which have negative dielectric anisotropy whose absolute values are large and that an excellent liquid crystal display device can be obtained by the use of the composition and thus have completed the present invention.

According to a first aspect of the present invention, there is provided a liquid crystal compound represented by the following general formula (3):

R¹-(A¹-X¹)ₐ₁-(A²-X²)ₐ₂-(A³-X³)ₐ₃-(A⁴-X⁴)ₐ₄-(A⁵) ₐ₅-Y¹ (3)

wherein R¹ and Y¹ each independently represents an alkyl group having 1 to 20 carbon atoms, a hydrogen atom, a halogen atom, or a cyano group, provided that at least one of the methylene groups present in the alkyl group, which are not adjacent to one another, may be substituted with an oxygen, sulfur or nitrogen atom, -C ≡C-, a silylene group wherein at least one hydrogen atom may be substituted with an alkyl group having 1 to 10 carbon atoms, or a vinylene group, or that at least one hydrogen atom of the alkyl group may be substituted with a fluorine or chlorine atom or that these alkyl groups may be optically active ones; X¹, X², X³ and X⁴ each independently represents a single bond, -(CH₂)₂-, -CH=CH-, -C ≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC ≡C-, -C≡CCH=CH-, or a group represented by the general formula (1) 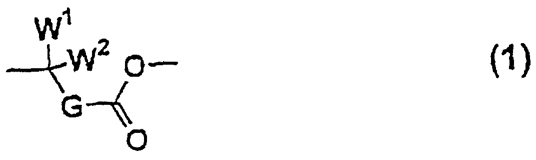 (wherein G represents an alkylene group having 1 to 3 carbon atoms wherein at least one hydrogen atom constituting the alkylene group may be substituted with a fluorine or chlorine atom; W¹ and W² each independently represents a hydrogen, fluorine or chlorine atom, provided that at least one of W¹ and W² represent a fluorine or chlorine atom), provided that at least one of the substituents X¹, X², X³ and X⁴ represent a group represented by Formula (1); the rings A¹, A², A³, A⁴ and A⁵ each independently represents a trans-cyclohexane-1,4-diyl, cyclohexane-1-en-1,4-diyl, 1,4-phenylene, bicyclo[1.1.1]pentane-1,3-diyl group, or a ring represented by the general formula (2) 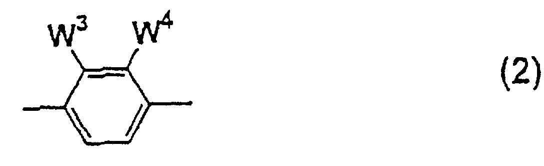 (wherein W³ and W⁴ each independently represents a fluorine or chlorine atom), provided that at least one of these rings A¹, A², A³, A⁴ and A⁵ represent a ring represented by Formula (2), that the carbon atoms constituting these rings may be substituted with a nitrogen, oxygen or sulfur atom and that each hydrogen atom present on the rings may be substituted with a halogen atom or a cyano group; a¹, a², a³, a⁴ and a⁵ each independently represents 0 or 1, provided that they satisfy the relation: 4 ≥ a¹+a²+a³+a⁴+a⁵ ≧ 2; and each atom constituting the compound may be substituted with its isotope.

More preferably, the group represented by Formula (1) is -CF₂CH₂COO- and that represented by Formula (2) is 2,3-difluorobenzene-1,4-diyl group.

According to a second aspect of the present invention, there is provided a liquid crystal composition which comprises at least two components including at least one liquid crystal compound defined above.

According to a third aspect of the present invention, there is provided a liquid crystal composition which comprises at least one liquid crystal compound defined above as a first component and at least one compound selected from the group consisting of those represented by the following general formulas (4), (5) and (6): 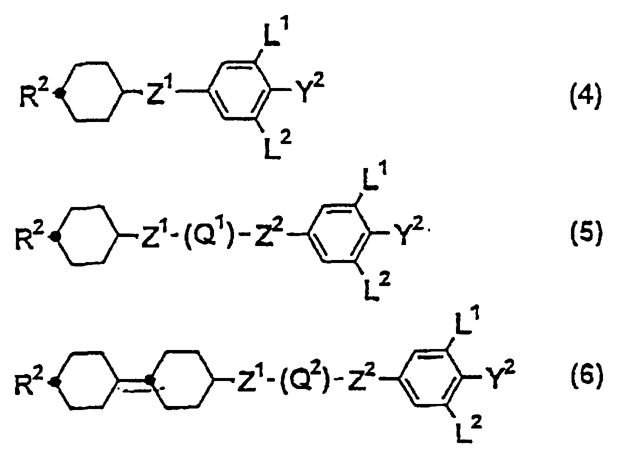 wherein R² represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y² represents a fluorine or chlorine atom, -OCF₃, -OCF₂H, -CF₃, -CF₂H, -CFH₂, -OCF₂CF₂H or -OCF₂CFHCF₃; L¹ and L² each independently represents a hydrogen or fluorine atom; Z¹ and Z² each independently represents -(CH ₂)₂-, -(CH₂)₄-, -COO-, -CF₂O-, -OCF₂-, -CH=CH- or a single bond; the ring Q¹ represents a trans-cyclohexane-1,4-diyl or 1,3-dioxane-2,5-diyl group, or a 1,4-phenylene group whose hydrogen atom may be substituted with a fluorine atom; the ring Q² represents a trans-cyclohexane-1,4-diyl group, or a 1,4-phenylene group whose hydrogen atom may be substituted with a fluorine atom; and each atom constituting the compound may be substituted with its isotope.

According to a fourth aspect of the present invention, there is provided a liquid crystal composition which comprises at least one liquid crystal compound defined above as a first component and at least one compound selected from the group consisting of those represented by the following general formulas (7) and (8) as a second component: 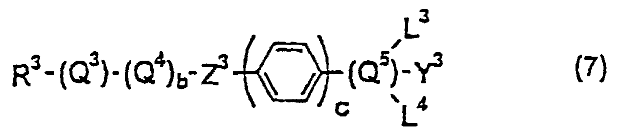 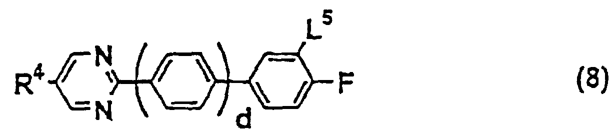 wherein R³ and R⁴ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y³ represents a cyano group or -C≡C-CN; the ring Q³ represents a trans-cyclohexane-1,4-diyl, 1,4-phenylene, 1,3-dioxane-2,5-diyl or pyrimidine-2,5-diyl group; the ring Q⁴ represents a trans-cyclohexane-1,4-diyl or pyrimidine-2,5-diyl group, or a 1,4-phenylene group whose hydrogen atom may be substituted with a fluorine atom; the ring Q⁵ represents a trans-cyclohexane-1,4-diyl or 1,4-phenylene group; Z³ represents -(CH₂)₂-, -COO- or a single bond; L³, L⁴ and L⁵ each independently represents a hydrogen or fluorine atom; b, c and d each independently represents 0 or 1; and each atom constituting the compound may be substituted with its isotope.

According to a fifth aspect of the present invention, there is provided a liquid crystal composition which comprises at least one liquid crystal compound defined above as a first component; at least one compound selected from the group consisting of those represented by the foregoing general formulas (4), (5) and (6) as a second component; and at least one compound selected from the group consisting of those represented by the following general formulas (9), (10) and (11) as a third component:

R⁵-(Q⁶)-Z⁴-(Q⁷)-Z⁵-R⁶ (9)

R⁵-(Q⁶)-Z⁴-(Q⁷)-Z⁵-(Q⁸)-R⁶ (10)

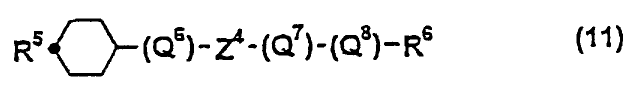

wherein R⁵ and R⁶ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁶, Q⁷ and Q⁸ each independently represents a trans-cyclohexane-1,4-diyl or pyrimidine-2,5-diyl group, or a 1, 4-phenylene group whose hydrogen atom may be substituted with a fluorine _{atom;} Z⁴ and Z⁵ each independently represents -C≡C-, -COO-, -(CH ₂)₂-, -CH=CH- or a single bond; and each atom constituting the compound may be substituted with its isotope.

According to a sixth aspect of the present invention, there is provided a liquid crystal composition which comprises at least one liquid crystal compound defined above as a first component and at least one compound selected from the group consisting of those represented by the following general formulas (12), (13) and (14) as a second component: 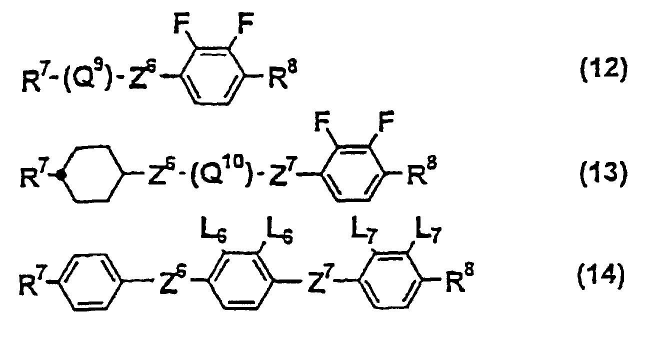 wherein R⁷ and R⁸ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁹ and Q¹⁰ each independently represents a trans-cyclohexane-1,4-diyl or 1,4-phenylene group; L⁶ and L⁷ each independently represents a hydrogen or fluorine atom, provided that L⁶ and L⁷ do not simultaneously represent a hydrogen atom; Z⁶ and Z⁷ each independently represents -(CH₂)₂-, -COO- or a single bond; and each atom constituting the compound may be substituted with its isotope.

According to a seventh aspect of the present invention, there is provided a liquid crystal composition which comprises at least one liquid crystal compound defined above as a first component; at least one compound selected from the group consisting of those represented by the foregoing general formulas (9), (10) and (11) as a second componen t; and at least one compound selected from the group consisting of those represented by the foregoing general formulas (12), (13) and (14) as a third component.

According to an eighth aspect of the present invention, there is provided a liquid crystal composition which comprises at least one liquid crystal compound defined above as a first component; at least one compound selected from the group consisting of those represented by the foregoing general formulas (7) and (8) as a second component; and at least one compound selected from the group consisting of those represented by the foregoing general formulas (9), (10) and (11) as a third component.

According to a ninth aspect of the present invention, there is provided a liquid crystal composition which comprises at least one liquid crystal compound defined above as a first component; at least one compound selected from the group consisting of those represented by the foregoing general formulas (4), (5) and (6) as a second component; at least one compound selected from the group consisting of those represented by the foregoing general formulas (7) and (8) as a third component; and at least one compound selected from the group consisting of those represented by the foregoing general formulas (9), (10) and (11) as a fourth component.

According to a tenth aspect of the present invention, there is provided a liquid crystal composition which comprises at least one optically active compound in addition to one of the liquid crystal compositions defined above.

According to an eleventh aspect of the present invention, there is provided a liquid crystal display device constituted by the use of one of the liquid crystal compositions defined above.

The sixth and seventh aspect of the present invention relate to N-type (having negative Δ ε) liquid crystal compositions. The N-type liquid crystal composition can be driven according to a variety of display modes such as vertically aligned mode and IPS mode as disclosed in J.P. KOKAI No. Hei 2-176625.

Moreover, the inventions according to the third, fourth, fifth, eighth and nineth aspects of the present invention relate to P-type (having positive Δ ε) liquid crystal compositions, but an N-type compound may also be used as a component for such P-type liquid crystal compositions. This permits not only arbitrary establishment of the dielectric anisotropy value of the liquid crystal composition depending on any particular use, but also the control of other characteristic properties of the composition such as the optical anisotropy value and the elastic constant.

Preferred embodiments of the compounds of the present invention, which carry the groups of Formula (1) and the rings of Formula (2) in the skeletal structures thereof will now be detailed below.

In Formula (1), G represents an alkylene group having 1 to 3 carbon atoms wherein at least one hydrogen atom constituting the alkylene group may be substituted with a fluorine or chlorine atom.

Specific examples of the groups represented by G in Formula (1) are as follows:
-CH₂-, -(CH₂)₂-, -(CH₂)₃-, -CHF-, -CHCl-, -CHFCH₂-, -CH₂CHF-, -(CHF)₂-, -CHClCH₂-, -CH₂CHCl-, -(CHCl)₂-, -CHClCHF-, -CHFCHCl-, -CHF(CH₂)₂-, -CH₂CHFCH₂-, -(CH₂)₂CHF-, -(CHF)₂CH₂-, -CHFCH₂CHF-, -CH₂(CHF)₂-, -(CHF)₃-, -CHClCH₂CH₂-, -CH₂CHClCH₂-, -CH₂CH₂CHCl-, -(CHCl)₂CH₂-, -CHClCH₂CHCl-, -CH₂(CHCl)₂-, -(CHCl)₃-, -CHFCHClCH₂-, -CH₂CHFCHCl-, -CHFCHClCHF-, -(CHF)₂CHCl-, and -CHFCH₂CHCl-.

In Formula (1), W¹ and W² each independently represents a hydrogen, fluorine or chlorine atom, provided that at least one of W¹ and W² represent a fluorine or chlorine atom.

Specific examples of the group represented by Formula (1) are as follows:
-CF₂CH₂COO-, -CF₂(CH₂)₂COO-, -CF₂(CH₂)₃COO-, -CF₂CHFCOO-, -CF₂CHClCOO-, -CCl₂CH₂COO-, -CCl₂(CH₂)₂COO-, -CCl₂(CH₂)₃COO-, -CCl₂CHFCOO-, -CCl₂CHClCOO-, -CF₂CHFCH₂COO-, -CF₂CH₂CHFCOO-, -CF₂(CHF)₂COO-, -CF₂CHClCH₂COO-, -CF₂CH₂CHClCOO-, -CF₂(CHCl)₂COO-, -CF₂CHClCHFCOO-, -CF₂CHFCHClCOO-, -CF₂CHF(CH₂)₂COO-, -CF₂CH₂CHFCH₂COO-, -CF₂(CH₂)₂CHFCOO-, -CF₂(CHF)₂CH₂COO-, -CF₂CHFCH₂CHFCOO-, -CF₂CH₂(CHF)₂COO-, -CF₂(CHF)₃COO-, -CF₂CHClCH₂CH₂COO-, -CF₂CH₂CHClCH₂COO-, -CF₂CH₂CH₂CHClCOO-, -CF₂(CHCl)₂CH₂COO-, -CF₂CHClCH₂CHClCOO-, -CF₂CH₂(CHCl)₂COO-, -CF₂(CHCl)₃COO-, -CF₂CHFCHClCH₂COO-, -CF₂CH₂CHFCHClCOO-, -CF₂CHFCHClCHFCOO-, -CF₂(CHF)₂CHClCOO-, and -CF₂CHFCH₂CHClCOO-.

Regarding the ring represented by Formula (2), W³ and W⁴ each independently represents a fluorine or chlorine atom.

Specific examples of the ring represented by Formula (2) are as follows:
2,3-Difluorobenzene-1,4-diyl, 2,3-dichlorobenzene-1,4-diyl, and 2-chloro-3-fluorobenzene-1,4-diyl.

Preferred embodiments of the compounds of the present invention, represented by Formula (3) will be described below.

The substituents R¹ and Y¹ of the compound of Formula (3) each independently represents an alkyl group having 1 to 20 carbon atoms, a hydrogen or halogen atom or a cyano group, provided that at least one of the methylene groups present in the alkyl group, which are not adjacent to each other, may be substituted with an oxygen, sulfur or nitrogen atom, a group -C ≡C-, a silylene group in which at least one hydrogen atom may be substituted with an alkyl group having 1 to 10 carbon atoms, or a vinylene group, that at least one of the hydrogen atoms present in the alkyl group may be substituted with a fluorine or chlorine atom or that these alkyl groups may be optically active ones.

Specific examples of the alkyl group represented by R¹ and Y¹ in Formula (3) include saturated alkyl groups simply consisting of carbon and hydrogen atoms, haloalkyl groups, alkoxy or haloalkoxy groups, alkenyl or haloalkenyl groups, alkenyloxy or haloalkenyloxy groups, alkynyl or haloalkynyl groups, alkynyloxy or haloalkynyloxy groups, alkoxyalkyl or haloalkoxyalkyl groups, alkoxyalkoxy or haloalkoxyalkoxy groups, alkanoyl or haloalkanoyl groups, alkanoyloxy or haloalkanoyloxy groups, and alkoxycarbonyl or haloalkoxycarbonyl groups.

Specific examples of R¹ and Y¹ are groups or atoms described below:
-CH₃, -C₂H₅, -C₃H₇, -C₄H₉, -C₅H₁₁, -C₆H₁₃, -C₇H₁₅, -C₈H₁₇, -C₉H₁₉, -C₁₀H₂₁, -CFH₂, -CF₂H, -CF₃, -CH₂CH₂F, -CH₂CHF₂, -CH₂CF₃, -CF₂CH₃, -CF₂CF₂H, -CHFCF₃, -CF₂CF₃, -(CH₂)₂CH₂F, -(CH₂)₂CHF₂, -(CH₂)₂CF₃, -CH₂CF₂CH₃, -CH₂CF₂CF₃, -CF₂C₂H₅, -C₃F₇, -CF₂CFHCF₃, -(CH₂)₃CH₂F, -(CH₂)₄CH₂F, -(CH₂)₅CH₂F, -CF₂C₃H₇, -CH₂CF₂C₂H₅, -(CH₂)₂CF₂CH₃, -CF₂C₄H₉, -CH₂CF₂CH₃, -(CH₂)₂CF₂C₂H₅, -(CH₂)₃CF₂CH₃, -OCH₃, -OC₂H₅, -OC₃H₇, -OC₄H₉, -OC₅H₁₁, -OC₆H₁₃, -OC₇H₁₅, -OC₈H₁₇, -OC₉H₁₉, -OC₁₀H₂₁, -OCFH₂, -OCF₂H, -OCF₃, -OCH₂CH₂F, -OCH₂CHF₂, -OCH₂CF₃, -OCF₂CH₃, -OCF₂CF₂H, -OCHFCF₃, -OCF₂CF₃, -O(CH₂)₂CH₂F, -O(CH₂)₂CHF₂, -O(CH₂)₂CF₃, -OCH₂CF₂CH₃, -OCH₂CF₂CF₃, -OCF₂C₂H₅, -OC₃F₇, -OCF₂CFHCF₃, -O(CH₂)₃CH₂F, -O(CH₂)₄CH₂F, -O(CH₂)₅CH₂F, -OCF₂C₃H₇, -OCH₂CF₂C₂H₅, -O(CH₂)₂CF₂CH₃, -OCF₂C₄H₉, -OCH₂CF₂CH₃, -O(CH₂)₂CF₂C₂H₅, -O(CH₂)₃CF₂CH₃, -CH=CH₂, -CH=CHCH₃, -CH=CHC₂H₅, -CH=CHC₃H₇, -CH=CHC₄H₉, -CH₂CH=CHCH₃, -CH₂CH=CHC₂H₅, -CH₂CH=CHC₃H₇, -CH₂CH=CHC₄H₉, -(CH₂)₂CH=CHCH₃, -(CH₂)₂CH=CHC₂H₅, -(CH₂)₂CH=CHC₃H₇, -(CH₂)₂CH=CHC₄H₉, -CH₂CH=CH₂, -(CH₂)₂CH=CH₂, -(CH₂)₃CH=CH₂, -(CH₂)₄CH=CH₂, -(CH₂)₅CH=CH₂, -CH=CHCH=CH₂, -CH=CHCH₂CH=CH₂, -CH=CH(CH₂)₂CH=CH₂, -CH=CH(CH₂)₃CH=CH₂, -CH₂CH=CH(CH₂)₂CH=CH₂, -(CH₂)₂CH=CH(CH₂)₂CH=CH₂, -CF=CF₂, -CH=CF₂, -CH=CHF, -CH₂CF=CF₂, -CH₂CH=CHF, -(CH₂)₂CH=CF₂, -(CH₂)₂CH=CHF, -(CH₂)₃CH=CF₂, -(CH₂)₃CH=CHF, -(CH₂)₄CH=CF₂, -(CH₂)₄CH=CHF, -(CH ₂)₅CH=CF₂, -(CH₂)₅CH=CHF, -CH=CHCH₂CH₂F, -CH=CHCH₂CH₂Cl, -CH=CHCH₂CHF₂, -CH=CHCH₂CF₃, -CH=CH(CH₂)₂CH₂F, -CH=CH(CH₂)₂CHF₂, -CH=CH(CH₂)₂CF₃, -CH=CH(CH₂)₃CH₂F, -CH=CH(CH₂)₃CHF₂, -CH=CH(CH₂)₃CF₃, -CH=CH(CH₂)₄CH₂F, -CH=CH(CH₂)₄CHF₂, -CH=CH(CH₂)₄CF₃, -CH₂CH=CH(CH₂)₂CH₂F, -CH₂CH=CHCH₂CH₂F, -(CH₂)₂CH=CH(CH₂)₂CH₂F, -(CH₂)₂CH=CHCH₂CH₂F, -CH=CHCH₂CH=CF₂, -CH=CH(CH₂)₂CH=CF₂, -CH=CH(CH₂)₂CF=CF₂, -CH=CH(CH₂)₃CHFCH₃, -OCH₂CH=CHCH₃, -OCH₂CH=CHC₂H₅, -OCH₂CH=CHC₃H₇, -OCH₂CH=CHC₄H₉, -O(CH₂)₂CH=CHCH₃, -O (CH₂)₂CH=CHC₂H₅, -O(CH₂)₂CH=CHC₃H₇, -O(CH₂)₂CH=CHC₄H₉, -OCH₂CH=CH₂, -O(CH₂)₂CH=CH₂, -O(CH₂)₃CH=CH₂, -O(CH₂)₄CH=CH₂, -O(CH₂)₅CH=CH₂, -OCH₂CH=CH(CH₂)₂CH=CH₂, -O(CH₂)₂CH=CH(CH₂)₂CH=CH₂, -OCH₂CF=CF₂, -OCH₂CH=CF₂, -OCH₂CF=CHF, -O(CH₂)₂CH=CF₂, -O(CH₂)₂CH=CHF, -O(CH₂)₃CH=CF₂, -O(CH₂)₃CH=CHF, -O(CH₂)₄CH=CF₂, -O(CH₂)₄CH=CHF, -O(CH₂)₅CH=CF₂, -O(CH₂)₅CH=CHF, -OCH₂CH=CHCH₂CH₂F, -O(CH₂)₂CH=CH(CH₂)₂CH₂F, -O(CH₂)₂CH=CHCH₂CH₂F, -C≡CH, -C ≡CCH₃, -C ≡CC₂H₅, -C ≡CC₃H₇, -C ≡CC₄H₉, -CH₂C≡CH, -CH₂C ≡CCH₃, -CH₂C≡ CC₂H₅, -CH₂C≡CC₃H₇, -CH₂C≡CC₄H₉, -(CH₂)₂C ≡ CH, -(CH₂)₂C≡CCH₃, -(CH₂)₂C≡CC₂H₅, -(CH₂)₂C ≡CC₃H₇, -(CH₂)₂C ≡CC₄H₉, -(CH₂)₃C ≡CH, -(CH₂)₃C ≡CCH₃, -(CH₂)₃C≡CC₂H₅, -(CH₂)₃C ≡CC₃H₇, -(CH₂)₃C ≡CC₄H₉, -C ≡CCF₃, -C≡CC₂F₅, -C ≡CC₃F₇, -CH₂C≡CCF₃, -(CH₂)₂C≡CCF₃, -OCH₂C ≡CH, -OCH₂C≡CCH₃, -OCH₂C≡CC₂H₅, -OCH₂C ≡CC₃H₇, -OCH₂C ≡CC₄H₉, -O (CH₂)₂C ≡CH, -O(CH₂)₂C ≡CC₃, -O(CH₂)₂C ≡CC₂H₅, -O(CH₂)₂C≡CC₃H₇, -O(CH₂)₂C≡CC₄H₉, -O(CH₂)₃C≡CH, -O(CH₂)₃C ≡CCH₃, -O(CH₂)₃C ≡CC₂H₅, -O(CH₂)₃C≡CC₃H₇, -O(CH₂)₃C≡CC₄H₉, -OCH₂C ≡CCF₃, -O(CH₂)₂C ≡CCF₃, -CH₂OCH₃, -CH₂OC₂H₅, -CH₂OC₃H₇, -CH₂OC₄H₉, -CH₂OC₅H₁₁, -(CH₂)₂OCH₃, -(CH₂)₂OC₂H₅, -(CH₂)₂OC₃H₇, -(CH₂)₂OC₄H₉, -(CH₂)₂OC₅H₁₁, -(CH₂)₃OCH₃, -(CH₂)₃OC₂H₅, -(CH₂)₃OC₃H₇, -(CH₂)₃OC₄H₉, -(CH₂)₃OC₅H₁₁, -(CH₂)₄OCH₃, -CF₂OCH₃, -CF₂OC₂H₅, -CF₂OC₃H₇, -CF₂OC₄H₉, -CF₂OC₅H₁₁, -CH₂OCF₃, -CH₂OC₂F₅, -OCH₂OCH₃, -OCH₂OC₂H₅, -OCH₂OC₃H₇, -OCH₂OC₄H₇, -OCH₂OC₅H₁₁, -O(CH₂)₂OCH₃, -O(CH₂)₂OC₂H₅, -O(CH₂)₂OC₃H₇, -O(CH₂)₂OC₄H₉, -O(CH₂)₂OC₅H₁₁, -O(CH₂)₃OCH₃, -O(CH₂)₃OC₂H₅, -O(CH₂)₃OC₃H₇, -O(CH₂)₃OC₄H₉, -O(CH₂)₃OC₅H₁₁, -O(CH₂)₄OCH₃, -OCH₂OCF₃, -OCH₂OC₂F₅, -COCF₃, -COCHF₂, -COC₂F₅, -COC₃F₇, -COCH₂CF₃, -OCOCH₃, -OCOC₂H₅, -OCOC₃H₇, -OCOC₅H₁₁, -OCOC₇H₁₅, -OCOCF₃, -OCOCHF₂, -OCOC₂F₅, -OCOC₃F₇, -OCOCH₂CF₃, -COOCH₃, -COOC₂H₅, -COOC₃H₇, -COOC₄H₉, -COOC₅H₁₁, -COOCH(CH₃)C₆H₁₃, -COOCH₂F, -COOCH₂CF₃, -COO(CH₂)₂CF₃, -COO(CH₂)₃CF₃, -COO(CH₂)₄CH₂F, hydrogen or halogen atoms, cyano or isocyano groups, and cyanate or isothiocyanate residues.

If paying attention, in particular, to the physicochemical stability of the compound of Formula (3), R¹ and Y¹ are preferably saturated alkyl groups simply consisting of carbon and hydrogen atoms, haloalkyl groups, alkoxy or haloalkoxy groups, alkoxyalkyl or haloalkoxyalkyl groups, alkoxyalkoxy or haloalkoxyalkoxy groups, alkanoyl or haloalkanoyl groups, alkanoyloxy or haloalkanoyloxy groups, alkoxycarbonyl or haloalkoxycarbonyl groups, hydrogen or halogen atoms or cyano groups.

If it is desired to further increase the absolute value of the negative dielectric anisotropy of the compound of Formula (3), R¹ and Y¹ are preferably substituents having a low polarity such as saturated alkyl groups simply consisting of carbon and hydrogen atoms, alkoxy groups, alkenyl groups, alkenyloxy groups, alkynyl groups, alkynyloxy groups, alkoxyalkyl groups, alkoxyalkoxy groups, alkanoyl groups, alkanoyloxy groups, alkoxycarbonyl groups, and hydrogen atom.

At least one of the substituents X¹, X², X³ and X⁴ present on the compound of Formula (3) is a group represented by Formula (1) and specific examples thereof include as follows:
-CF₂CH₂COO-, -CF₂(CH₂)₂COO-, -CF₂(CH₂)₃COO-, -CF₂CHFCOO-, -CF₂CHClCOO-, -CCl₂CH₂COO-, -CCl₂(CH₂)₂COO-, -CCl₂(CH₂)₃COO-, -CCl₂CHFCOO-, -CCl₂CHClCOO-, -CF₂CHFCH₂COO-, -CF₂CH₂CHFCOO-, -CF₂(CHF)₂COO-, -CF₂CHClCH₂COO-, -CF₂CH₂CHClCOO-, -CF₂(CHCl)₂COO-, -CF₂CHClCHFCOO-, -CF₂CHFCHClCOO-, -CF₂CHF(CH₂)₂COO-, -CF₂CH₂CHFCH₂COO-, -CF₂(CH₂)₂CHFCOO-, -CF₂(CHF)₂CH₂COO-, -CF₂CHFCH₂CHFCOO-, -CF₂CH₂(CHF)₂COO-, -CF₂(CHF)₃COO-, -CF₂CHClCH₂CH₂COO-, -CF₂CH₂CHClCH₂COO-, -CF₂CH₂CH₂CHClCOO-, -CF₂(CHCl)₂CH₂COO-, -CF₂CHClCH₂CHClCOO-, -CF₂CH₂(CHCl)₂COO-, -CF₂(CHCl)₃COO-, -CF₂CHFCHClCH₂COO-, -CF₂CH₂CHFCHClCOO-, -CF₂CHFCHClCHFCOO-, -CF₂(CHF)₂CHClCOO-, and -CF₂CHFCH₂CHClCOO-.

Preferred substituents X¹, X², X³ and X⁴ other than the foregoing are as follows: a single bond, -(CH₂)₂-, -CH=CH-, -C≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC ≡ C-, and -C≡CCH=CH-.

If paying attention, in particular, to the physicochemical stability of the compound of Formula (3), preferred substituents X¹, X², X³ and X⁴ are those represented by Formula (1) (such as -CF₂CH₂COO-), but preferred examples of the substituents X¹, X², X³ and X⁴ other than those represented by Formula (1) include as follows:

Single bond, -(CH₂)₂-, -COO-, -OCO-, -CH₂O-, -OCH₂-, -(CH₂)₄-, -(CH₂)₃O-, -O(CH₂)₃-, -CF₂O-, -OCF₂-, -CH₂CF₂-, -CF₂CH₂-, -(CF₂)₂-, -(CF₂)₄-, -(CH₂)₂COO-, and -OCO(CH₂)₂-.

If it is desired to further increase the absolute value of the negative dielectric anisotropy of the compound of Formula (3), preferred substituents X¹, X², X³ and X⁴ are those represented by Formula (1) (such as -CF₂CH₂COO-), but preferred examples of the substituents X¹, X², X³ and X⁴ other than those represented by Formula (1) include as follows:

Single bond, -(CH₂)₂-, -COO-, -OCO-, -CH₂O-, -OCH₂-, -(CH₂)₄-, -(CH₂)₃O-, -O(CH₂)₃-, -CF₂O-, -OCF₂-, -CH₂CF₂-, -CF₂CH₂-, -(CF₂)₂-, -(CF₂)₄-, -(CH₂)₂COO-, and -OCO(CH₂)₂-.

At least one of the rings A¹, A², A³, A⁴ and A⁵ of the compound represented by Formula (3) is a ring represented by Formula (2). Specific examples thereof are those listed below:
2,3-Difluorobenzene-1,4-diyl, 2,3-dichlorobenzene-1,4-diyl, and 2-chloro-3-fluorobenzene-1,4-diyl.

Preferred rings other than the foregoing are, for instance, bicyclo[1.1.1]pentane-1,3-diyl, trans-cyclohexane-1,4-diyl, cyclohexa-1-en-1,4-diyl and 1,4-phenylene, provided that the carbon atoms constituting these rings may be substituted with a nitrogen, oxygen or sulfur atom and that the hydrogen atoms present on the rings may likewise be substituted with a halogen atom or a cyano group.

Specific examples thereof include 1-cyano-trans-cyclohexane-1,4-diyl, 2,2-difluoro -trans-cyclohexane-1,4-diyl, 2-fluorocyclohexa-1-en-1,4-diyl, and 2,2-dichlorobicyclo[1.1.1] pentane-1,3-diyl.

Examples of the rings whose hydrogen atoms present thereon may be substituted with a fluorine or chlorine atom or a cyano group are as follows:

Bicyclo[1.1.1]pentane-1,3-diyl, trans-cyclohexane-1,4-diyl, cyclohexa-1-en-1,4-diyl, 1,4-phenylene, 1,3-dioxane-2,5-diyl, pyrimidine-2,5-diyl, pyridine-2,5-diyl, pyrazine-2,5-diyl, pyridazine-3,6-diyl, 1,3-dithian-2,5-diyl, 1,3-oxathian-2,5-diyl, 2,3-difluorobenzene-1,4-diyl, and bicyclo[2.2.2]octane-1,4-diyl.

If paying attention, in particular, to the physicochemical stability of the compound of Formula (3), preferred rings A¹, A², A³, A⁴ and A⁵ of the compound represented by Formula (3) include those represented by Formula (2) (such as 2,3-difluorobenzene-1,4-diyl), but preferred rings A¹, A², A³, A⁴ and A⁵ other than the foregoing are as follows:

1-cyano-trans-cyclohexane-1,4-diyl, 2,2-difluoro-trans-cyclohexane-1,4-diyl, 2-fluorocyclohexa-1-en-1,4-diyl, and 2,2-dichlorobicyclo [1.1.1]pentane-1,3-diyl.

Examples of the rings whose hydrogen atoms present thereon may be substituted with a fluorine or chlorine atom or a cyano group are as follows:
Bicyclo[1.1.1]pentane-1,3-diyl, trans-cyclohexane-1,4-diyl, cyclohexa-1-en-1,4-diyl, 1,4-phenylene, 1,3-dioxane-2,5-diyl, pyrimidine-2, 5-diyl, pyridine-2,5-diyl, pyridazine-3,6-diyl, 1,3-dithian-2,5-diyl, 2,3-difluorobenzene-1,4-diyl, and 1,3-oxathian-2,5-diyl.

If it is desired to further increase the absolute value of the negative dielectric anisotropy of the compound of Formula (3), preferred rings A¹, A², A³, A⁴ and A⁵ are those represented by Formula (2) (such as 2,3-difluorobenzene-1,4-diyl), but preferred rings A¹, A², A³, A⁴ and A⁵ other than those listed above are as follows:
1-cyano-trans-cyclohexane-1,4-diyl, 2,2-difluoro-trans -cyclohexane-1,4-diyl, 2-fluorocyclohexa-l-en-1,4-diyl, and 2,2-dichlorobicyclo [1.1.1]pentane-1,3-diyl.

Examples of the rings whose hydrogen atoms present thereon may be substituted with a fluorine or chlorine atom or a cyano group are as follows:
Trans-cyclohexane-1,4-diyl, cyclohexa-1-en-1,4-diyl, and pyridazine-3,6-diyl.

The compounds according to the first aspect of the present invention and preferred embodiments thereof have negative dielectric anisotropy values whose absolute values are large. Therefore, a liquid crystal composition having a negative dielectric anisotropy value can be prepared by mainly using the foregoing compounds. This liquid crystal composition is useful in the preparation of display devices which make use of a liquid crystal composition having negative dielectric anisotropy, including, for instance, vertically aligned mode and IPS mode as disclosed in J.P. KOKAI No. Hei 2-176625. Moreover, the dielectric anisotropy value can appropriately be established by the use of a mixture of the compound of the present invention with other liquid crystal compounds or compositions to thus expand the area of applications of these other liquid crystal compounds or compositions.

The compounds according to the first aspect of the present invention and preferred embodiments thereof have small elastic constants and the temperature-dependency of the constants is also low. Therefore, the use of the compounds of the present invention would permit the preparation of novel liquid crystal compositions having a low driving voltage.

The compounds according to the first aspect of the present invention and preferred embodiments thereof exhibit good miscibility with other liquid crystal compounds. For this reason, if the compounds of the invention are incorporated into liquid crystal compositions, any problem such as separation of compounds hardly arises. In addition, a large amount of the compound of the present invention can be incorporated into a liquid crystal composition and accordingly, the excellent characteristics of the compound of the invention can intensively be reflected in the resulting liquid crystal composition.

The compounds according to the first aspect of the present invention and preferred embodiments thereof permit appropriate design of the structure of the compounds such that they have a desired level of the optical anisotropy value. In other words, the compound should be desighed so as to have rings including a large number of sites carrying resonance structures such as aromatic rings, if it is necessary to use a compound having a particularly high optical anisotropy value. On the other hand, if it is necessary to use a compound having a low optical anisotropy value, the compound should be desighed so as to have rings including a large number of sites free of any resonance structure such as trans-cyclohexane-1,4-diyl ring.

If describing this more fully, the compound of Formula (3) according to the present invention may have a desired level of the optical anisotropy value by appropriately selecting the substituents R¹, Y¹, X¹, X², X³, X⁴, A¹, A², A³, A⁴, A⁵, a¹, a², a³, a⁴ and a⁵. More specifically, the optical anisotropy value may be controlled by appropriately selecting the rings A¹, A², A³, A⁴ and A⁵. In other words, the selection of a ring or rings each including a large number of sites carrying resonance structures such as aromatic rings permits the preparation of a compound having particularly high optical anisotropy, while the selection of a ring or rings each including a large number of sites free of any resonance structure such as trans-cyclohexane-1,4-diyl ring permits the preparation of a compound having low optical anisotropy.

The degree of freedom in the design of a liquid crystal panel would widely be expanded by arbitrarily selecting the optical anisotropy value.

All of the compounds according to the first aspect of the present invention and preferred embodiments thereof have low viscosities and therefore, they do not undesirably increase the overall viscosity of the resulting liquid crystal composition even if a large amount of the compound is incorporated into the composition. In addition, the compound of the invention exhibits very low temperature-dependency of the viscosity, in particular, at a low temperature. The use of the liquid crystal compound having such an excellent viscosity permits the preparation of a liquid crystal composition having high speed responsibility.

All of the compounds according to the first aspect of the present invention and preferred embodiments thereof are physicochemically stable and accordingly, the liquid crystal compositions prepared using them have high specific resistance and a high voltage-holding ratio. In other words, the compounds are highly stable against external factors such as ultraviolet rays and heat and have physicochemical stability sufficient for use as ingredients of practically used liquid crystal compositions.

The phase transition temperature of the compounds according to the first aspect of the present invention and preferred embodiments thereof can be controlled by appropriately selecting the number of rings attached to each compound. In other words, it would be sufficient to select a compound carrying not less than 4 rings when it is desired for the achievement of a particularly high clearing point, a compound having 3 rings when it is desired for the achievement of a medium clearing point, and a compound having 2 rings when it is desired for the achievement of a particularly low clearing point.

If describing this more fully, the phase transition points of the compounds of Formula (3) according to the present invention can be controlled by appropriately selecting the values of a¹, a², a³, a⁴ and a⁵. More specifically, it would be sufficient to select a compound carrying not less than 4 rings and a¹+a²+a³+a⁴+a⁵≧4 when it is desired for the achievement of a particularly high clearing point; a compound having 3 rings and a¹+a²+a³+a⁴+a⁵=3 when it is desired for the achievement of a medium clearing point; and a compound having 2 rings and a¹+a²+a³+a⁴+a⁵=2 when it is desired for the achievement of a particularly low clearing point.

As has been discussed above, the compounds of the first aspect of the present invention and the preferred embodiments thereof have various characteristic properties favorable for use as electro-optical display materials. The use of the compounds of the invention permits the preparation of novel liquid crystal compositions having good characteristics. In addition, the present invention also permits the production of liquid crystal compounds having desired properties by appropriately designing the structure thereof depending on each particular use as well as liquid crystal composition and liquid crystal display devices using the compounds.

For instance, the compound of the present invention is characterized by having a large negative dielectric anisotropy value, but the compound can not only be used in a liquid crystal composition having a negative dielectric anisotropy value, but also can be used to control the dielectric anisotropy value and other characteristics of a composition having a positive dielectric anisotropy value by the addition of the former to the latter.

If describing this more fully, the compound of the invention and the liquid crystal composition including the same can be used in various display modes which make use of compounds or compositions having negative dielectric anisotropy values (such as the vertically aligned modes as disclosed in J.P. KOKAI No. Hei 2-176625, IPS, ECB (for instance, HAN or DAP), DS, GH or PC), in particular, the vertically aligned modes as disclosed in J.P. KOKAI No. Hei 2-176625 and IPS. In addition to the foregoing, the compound and composition of the invention can also be used for the improvement or control of various characteristics (for instance, dielectric anisotropy, elastic constant, optical anisotropy, viscosity, and/or physicochemical stability) of liquid crystal compositions used for various display modes (such as TN, STN, or TN-based AM (e.g., TFT or MIM) modes) which make use of compounds or compositions having positive dielectric anisotropy values.

The liquid crystal composition of the present invention will now be detailed below. The liquid crystal composition of the invention preferably comprises 0.1 to 99.9% by weight of at least one compound as defined in the first aspect of the present invention and the preferred embodiments thereof in order to impart excellent characteristic properties to the composition.

More specifically, the liquid crystal composition of the present invention comprises a compound selected from the group consisting of those represented by Formulas (4) to (14) in an appropriate amount depending on each particular purpose, in addition to the compound of the present invention as a first component.

The following are examples of preferred compounds represented by Formulas (4) to (6) which can be used in the liquid crystal composition of the invention (in these compounds, R² and Y² are the same as those defined above):

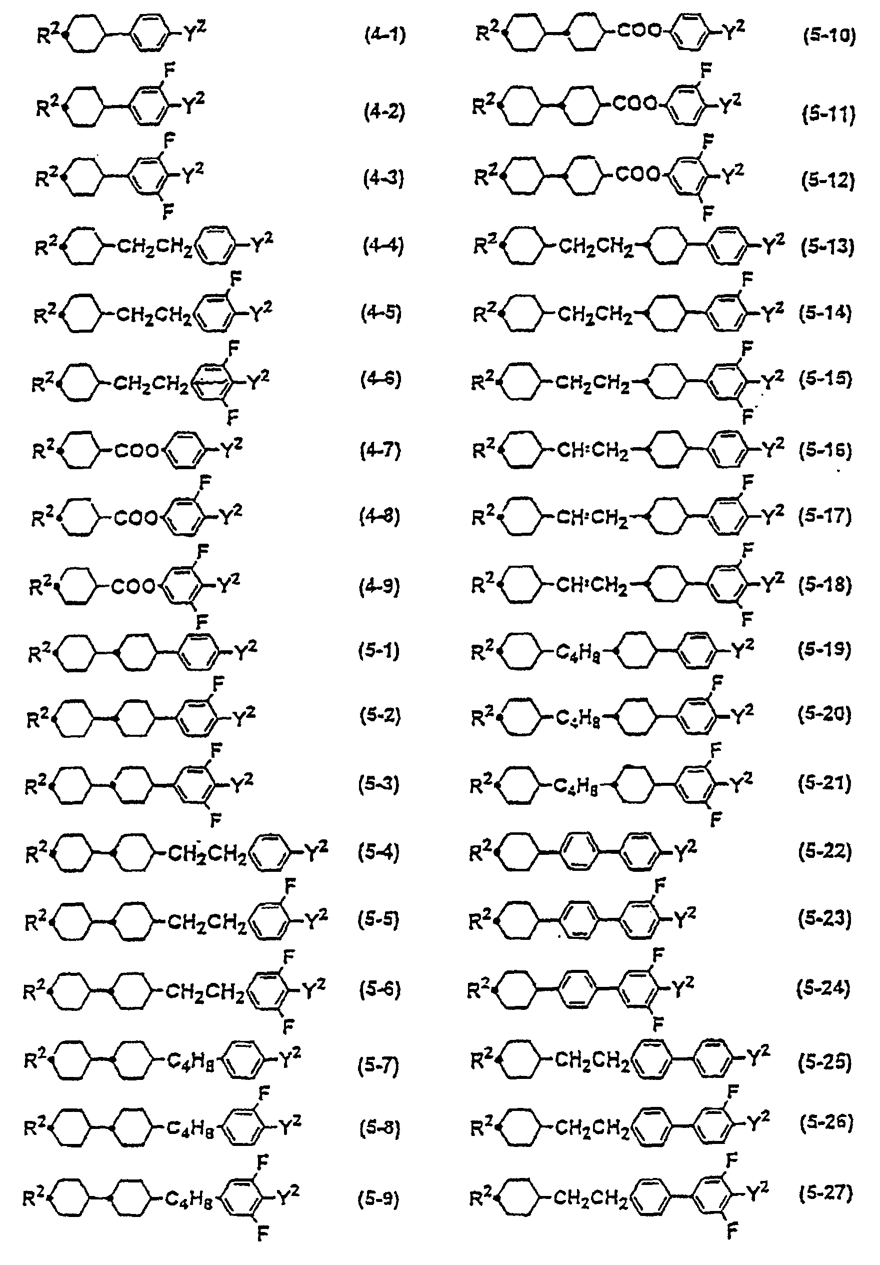

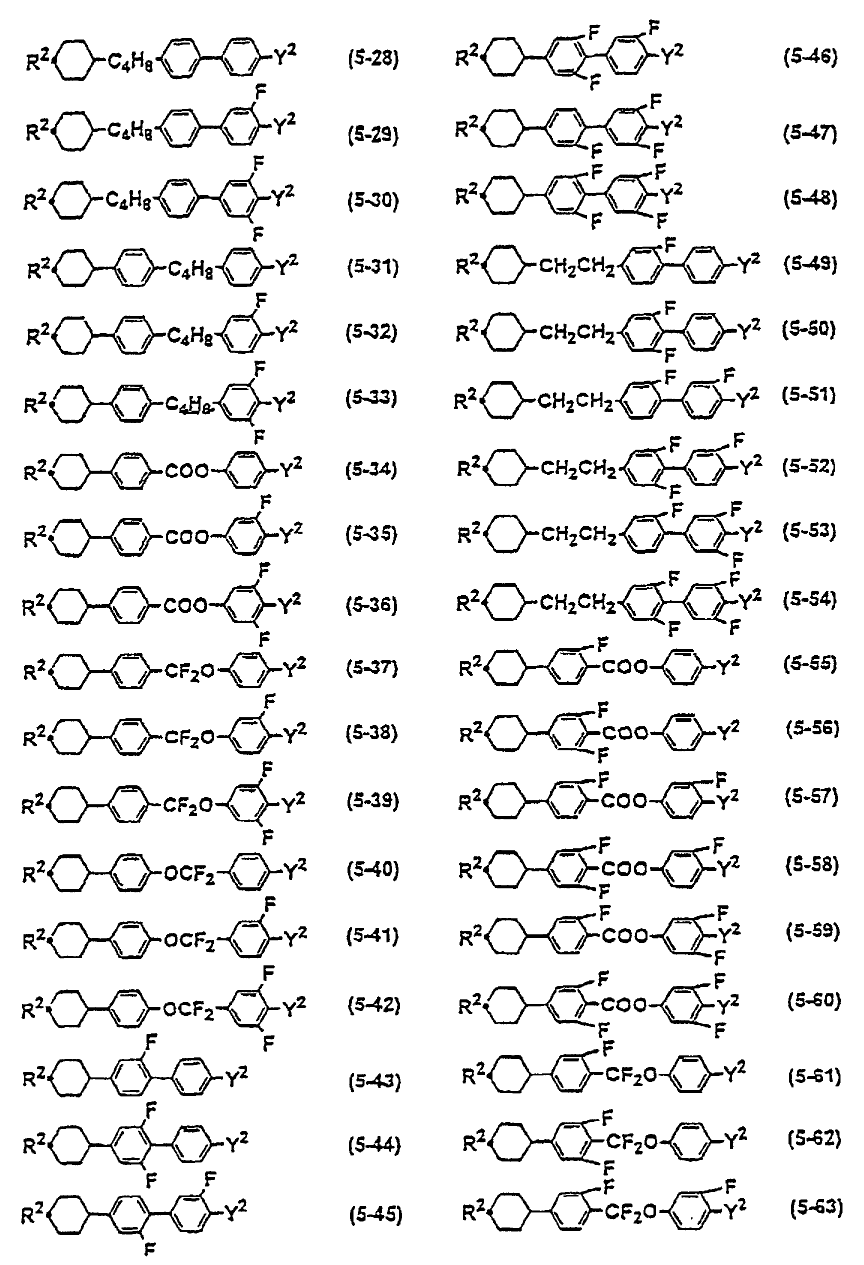

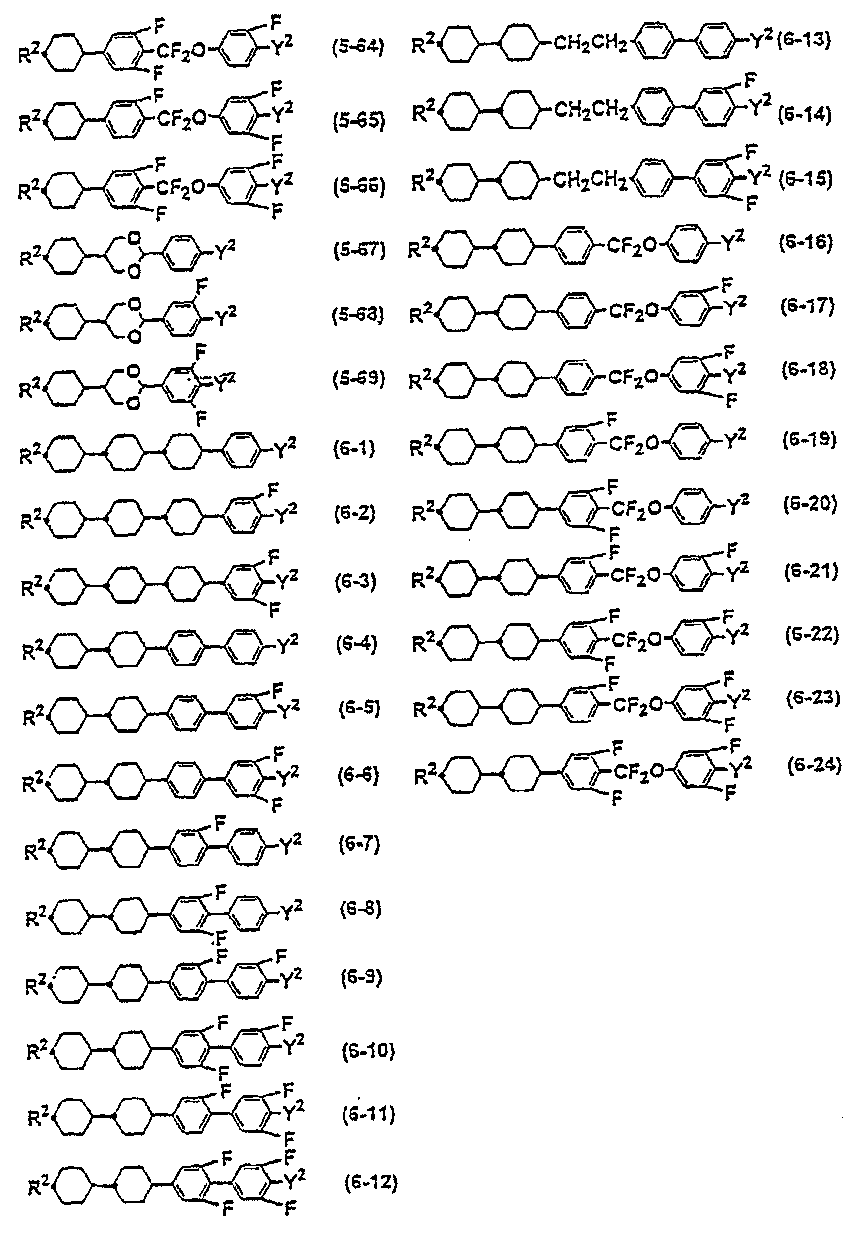

The compounds represented by Formulas (4) to (6) have positive dielectric anisotropy values, are quite excellent in thermal and chemical stability and are particularly preferably used when preparing liquid crystal compositions used in TFT's which require high reliability such as a high voltage-holding ratio or a high specific resistance.

The amount of the compounds of Formulas (4) to (6) used when preparing a liquid crystal composition used in TFT's ranges from 0.1 to 99.9% by weight, preferably 10 to 97% by weight and more preferably 40 to 95% by weight based on the total weight of the composition. In addition, the liquid crystal composition may further comprise the compounds of Formulas (9) to (11) in order to control the viscosity of the composition.

The compounds represented by Formulas (4) to (6) can likewise be used when preparing a liquid crystal composition for STN' or TN's. In this case, they are preferably used in an amount of not more than 5 0% by weight.

The following are examples of preferred compounds represented by Formulas (7) to (8) which can be used in the liquid crystal composition of the invention (in these compounds, R³, Y³ and R⁴ are the same as those defined above):

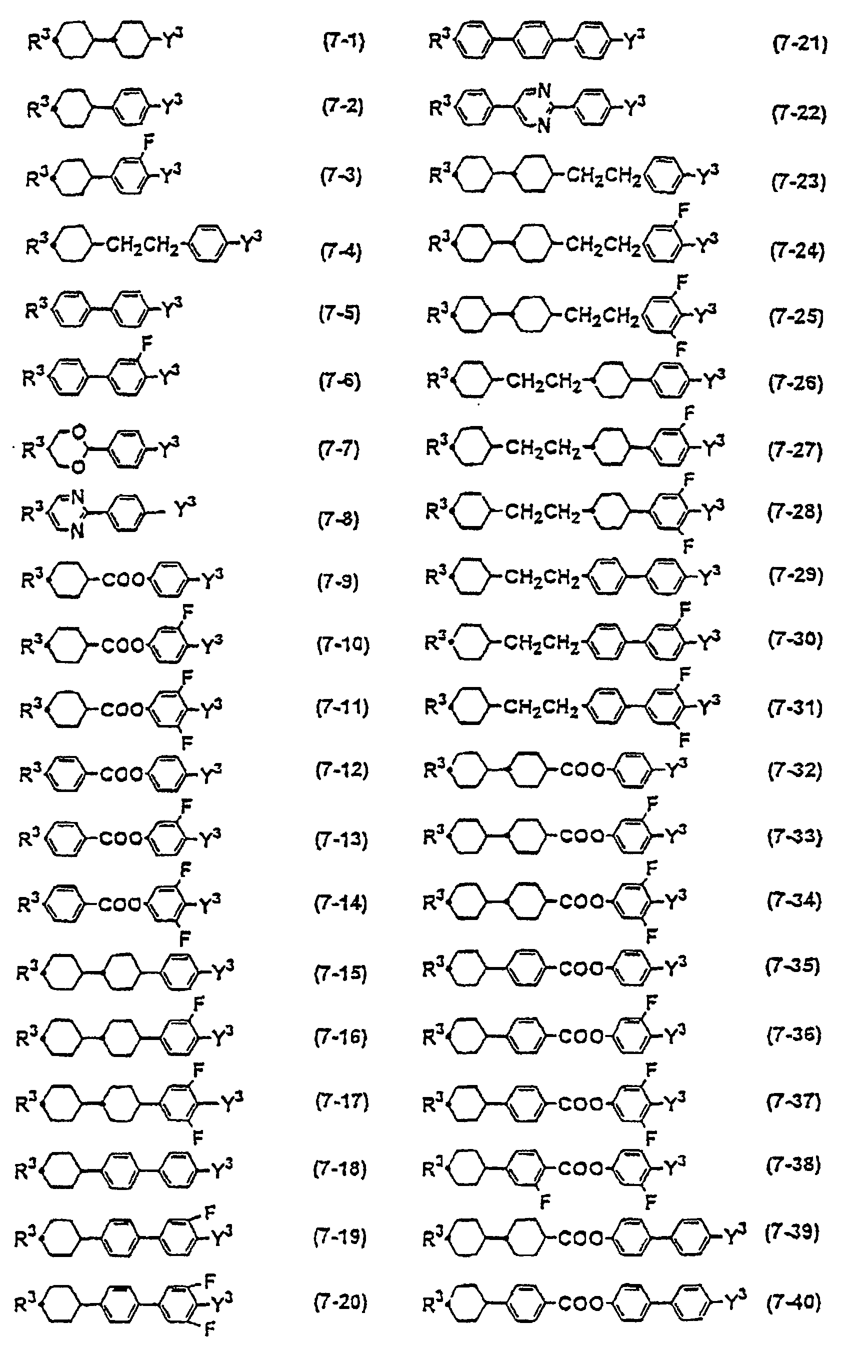

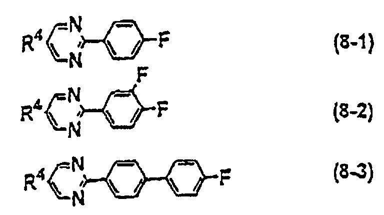

The compounds represented by Formulas (7) to (8) have large and positive dielectric anisotropy values and are used, in particular, for the purpose of reducing the threshold voltage of the resulting liquid crystal composition. Moreover, they can be used for controlling the optical anisotropy value and for expanding the nematic range, for instance, increasing the clearing point. Further they are also used for the improvement in the steepness of the V-T curve of the liquid crystal composition for STN's or TN's.

The compounds represented by Formulas (7) to (8) are preferred, in particular, when preparing the liquid crystal composition for STN's or TN's.

If the amount of the compound of Formula (7) or (8) used increases, the threshold voltage of the resulting liquid crystal composition is reduced and the viscosity thereof increases. Therefore, it is advantageous to use the compounds in a large amount since the resulting mode can be operated at a low voltage, so far as the composition satisfies the requirement for viscosity. If preparing a liquid crystal composition for STN's or TN's, the amount of the compound of Formula (7) or (8) ranges from 0.1 to 99.9% by weight, preferably 10 to 97% by weight and more preferably 40 to 95% by weight based on the total weight of the composition.

The following are examples of preferred compounds represented by Formulas (9) to (11) which can be used in the liquid crystal composition of the invention (in these compounds, R⁵ and R⁶ are the same as those defined above):

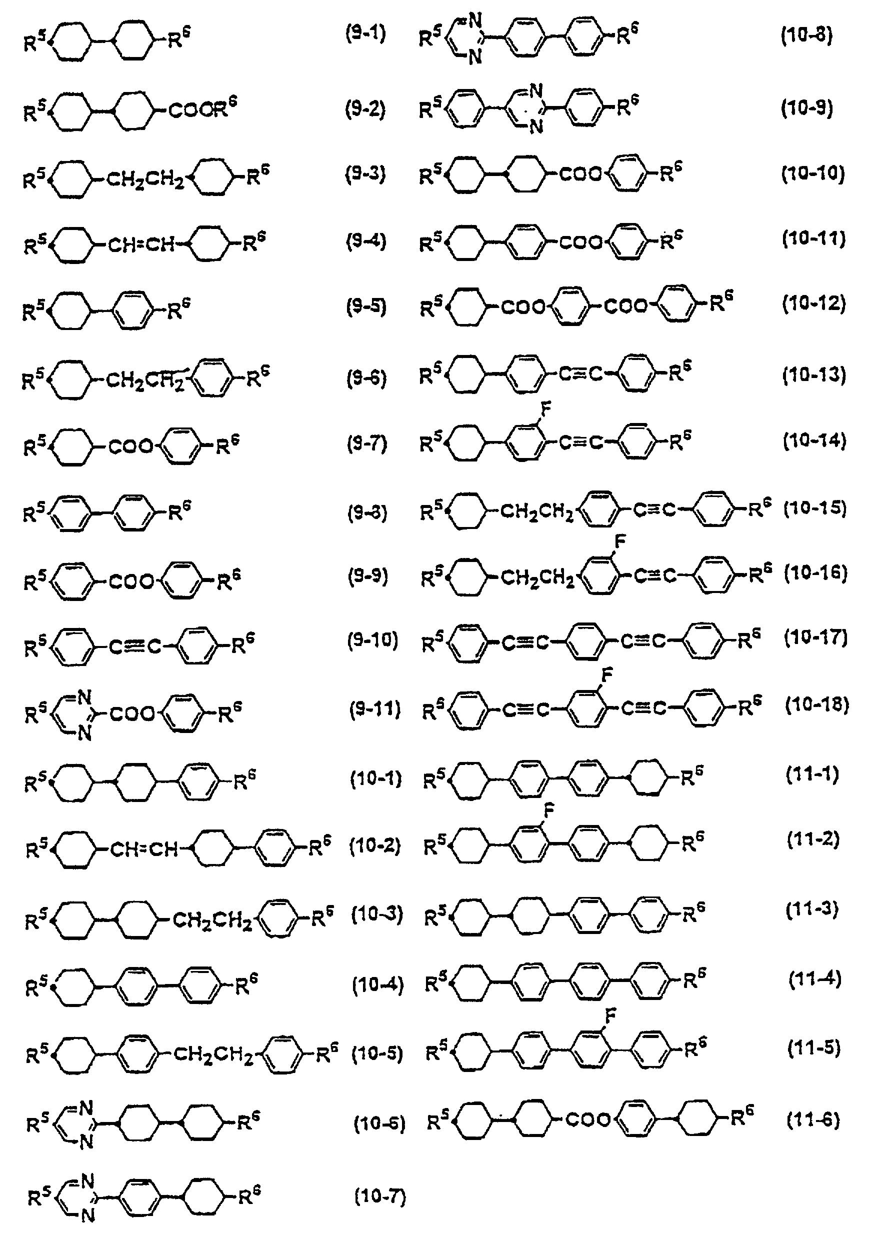

The compounds represented by Formulas (9) to (11) have dielectric anisotropy values whose absolute values are small and are almost neutral ones. The compounds of Formula (9) are mainly used for controlling the viscosity or optical anisotropy value of the resulting liquid crystal composition, while the compounds of Formula (10) and (11) are used for expanding the nematic range, for instance, increasing the clearing point or for controlling the optical anisotropy value.

If the amount of the compounds of Formulas (9) to (11) used increases, the threshold voltage of the resulting liquid crystal composition increases and the viscosity thereof decreases. Therefore, they can be used in a large amount inasmuch as the composition satisfies the requirememt for threshold voltage. When preparing a liquid crystal composition for TFT's, the amount of the compounds of Formulas (9) to (11) is preferably not more than 40% by weight and more preferably not more than 35% by weight based on the total weight of the composition, while when preparing a liquid crystal composition for STN's or TN's, the amount thereof is preferably not more than 70% by weight and more preferably not more than 60% by weight based on the total weight of the composition.

The following are examples of preferred compounds represented by Formulas (12) to (14) which can be used in the liquid crystal composition of the invention (in these compounds, R⁷ and R⁸ are the same as those defined above):

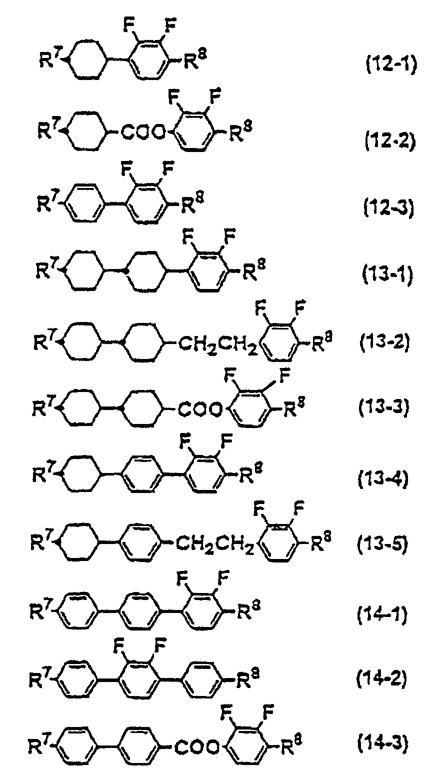

The compounds represented by Formulas (12) to (14) have negative dielectric anisotropy values like the compounds according to the first aspect of the present invention and the preferred embodiments thereof and are used as base compounds for N-type liquid crystal compositions or are used for controlling the dielectric anisotropy of P-type liquid crystal compositions.

Moreover, the compounds of Formula (12) are bicyclic compounds and mainly used for controlling the threshold values, viscosities or refractive index values of the resulting compositions. The compounds of Formula (13) are used for expanding the nematic range, for instance, increasing the clearing point or for controlling the optical anisotropy value. Further the compounds of Formula (14) are used for expanding the nematic range, for reducing the threshold voltage and for increasing the refractive index value of the resulting liquid crystal composition.

The compounds of Formula (12) to (14) are mainly used in the N-type liquid crystal compositions and if the amount thereof used increases, the threshold value of the resulting liquid crystal composition is reduced, while the viscosity thereof is increased. For this reason, these compounds are desirably used in a small amount inasmuch as the liquid crystal composition satisfies the requirement for threshold voltage. However, the absolute value of the negative dielectric anisotropy thereof is not more than 5 and therefore, if the amount thereof used is less than 40% by weight, the resulting display device cannot sometimes be operated at a low voltage. When preparing a liquid crystal composition for N-type TFT's, the amount of the compounds of Formula (12) to (14) is preferably not less than 40% by weight and more preferably 50 to 95% by weight based on the total weight of the composition. Moreover, the compounds of Formula (12) to (14) are often incorporated into P-type compositions for controlling the elastic constant and the voltage-transmittance curve (V-T curve) of the resulting liquid crystal composition. In this case, the amount of the compounds of Formula (1 2) to (14) is preferably not more than 30% by weight based on the total weight of the composition.

In addition, the liquid crystal composition of the invention may in general comprise an optically active compound in order to induce the formation of a helical structure of the liquid crystal composition to thus achieve a desired twist angle and prevent the occurrence of any reverse twist, except for some particular cases such as liquid crystal compositions for OCB (Optically Compensated Birefringence).

Any known optically active compounds used for such purposes may be used in the present invention, but preferred are optically active compounds listed below:

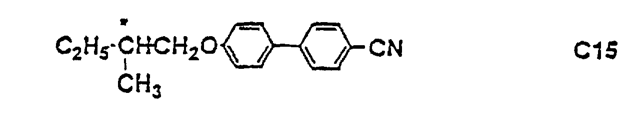

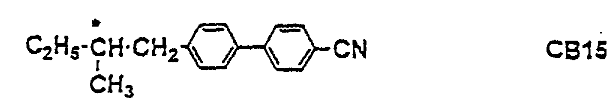

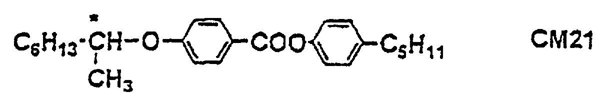

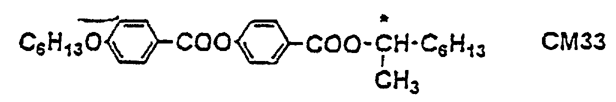

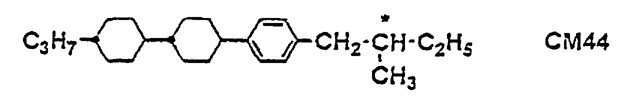

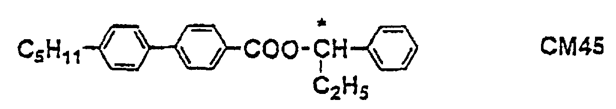

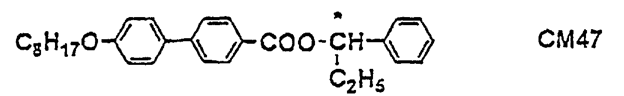

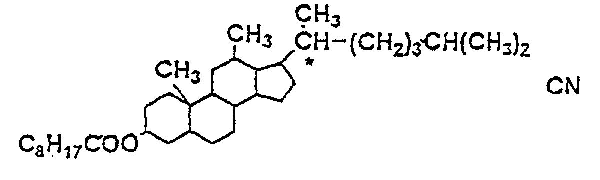

These optically active compounds are usually incorporated into the liquid crystal composition of the invention to thus control the pitch of twist. The pitch of twist is preferably adjusted to the range of from 40 to 200 µm in case of the liquid crystal compositions for TF T's and TN's. Moreover, it is adjusted to the range of from 6 to 20 µm in case of the liquid crystal compositions for STN's and 1.5 to 4 µm for bistable TN's. Moreover, at least two optically active compounds may be added to the composition to control the temperature-dependency of the pitch.

The liquid crystal composition of the present invention may be used as the composition for GH's by addition of a dichroic dye such as merocyanine, styryl, azo, azomethine, azoxy, quinophthalone, anthraquinone, or tetrazine type ones. Alternatively, the composition of the present invention can likewise be used as a liquid crystal composition for polymer-dispersion type liquid crystal display devices (PDLCD) including NCAP' which are produced by encapsulation of a nematic liquid crystal and polymer network liquid crystal display devices (PNLCD) produced by forming a three-dimensional network polymer in the liquid crystal. Moreover, the liquid crystal composition of the invention may be used in DS's.

The composition of the invention can be prepared by the commonly used methods. In general, it is prepared by a method wherein various ingredients are dissolved together at a high temperature.

The compounds according to the first aspect of the present invention and the preferred embodiments thereof can easily be prepared by any known organic chemical synthesis technique. An example of the production methods will be given in the following reaction scheme 1:

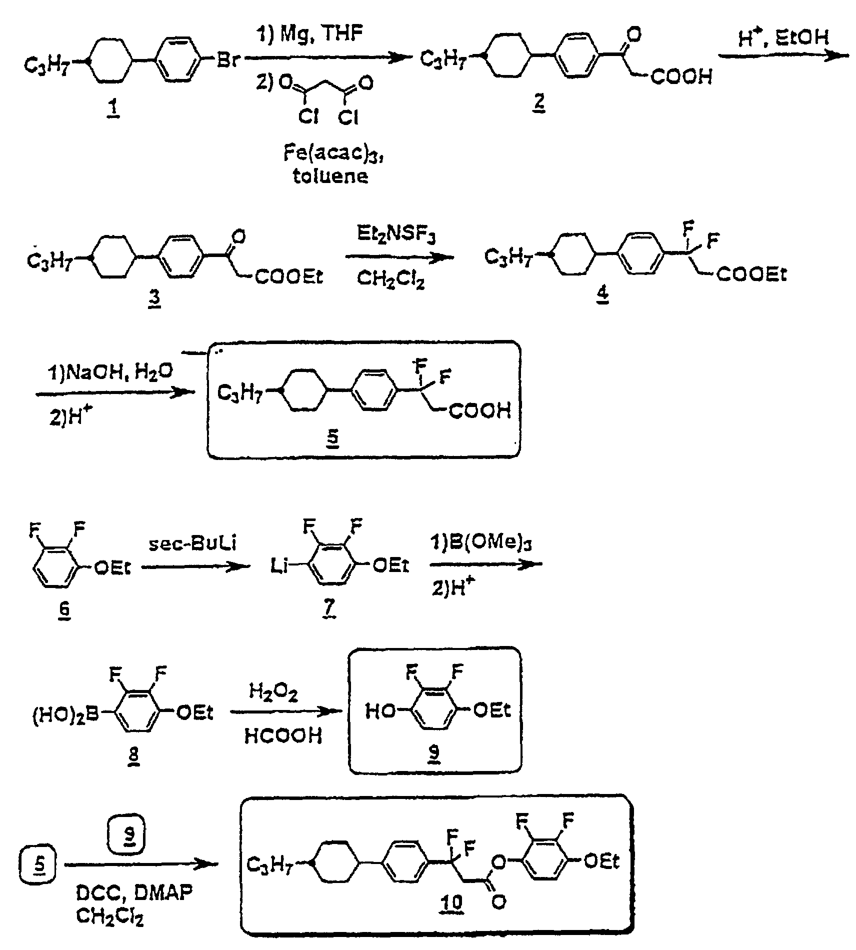

The foregoing route of synthesis will be detailed below.

The compound represented by Formula 1 is reacted with magnesium in THF (tetrahydrofuran), followed by reacting the resulting Grignard reagent with malonic acid chloride in the presence of iron(III) acetylacetone complex in toluene to give a compound represented by Formula 2 and then reacting the compound with ethanol in the presence of an acid catalyst to give the ester of Formula 3. Thereafter, the carbonyl group of the compound of Formula 3 is fluorinated using DAST (Et₂NSF₃) in methylene chloride (CH₂Cl₂) to give the compound of Formula 4 followed by hydrolyzation of the ester group with an alkali to give the carboxylic acid of Formula 5.

The compound of Formula 5 is converted into a lithium derivative (Formula 7) with sec-butyl lithium, followed by reacting the derivative with trimethyl borate in THF to give the compound of Formula 8 and oxidation thereof with hydrogen peroxide to give the compound of Formula 9.

The compounds of Formula 5 and 9 are reacted with DCC (dicyclohexyl carbodiimide) in the presence of DMAP (4-(N,N-dimethylamino)pyridine) in methylene chloride to give a desired compound of Formula 10.

The method of preparing the compound of the present invention and the use thereof will be described below in detail with reference to the following working Examples, but the present invention is not restricted to these specific Examples at all. In the description of the following Examples, the symbol N represents the-equivalent concentration (gram eq./l); M represents the molar concentration (mol/l); Δ ε (dielectric anisotropy value) of a liquid crystal compound represents the value obtained by extrapolating the values (as measured at 25 °C) observed for the compositions comprising 85 wt% of the following mother liquid crystal A and 15 wt% of each corresponding compound; and Δn (optical anisotropy value) represents the measured value (as determined at 25 °C) for the composition comprising 85 wt% of the following mother liquid crystal B and 15 wt% of each corresponding compound.

### Composition of Mother Liquid Crystal A

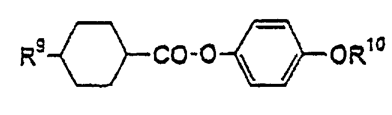

Five kinds of ester compounds represented by the foregoing general formula and whose both terminal alkyl groups (R⁹, R¹⁰) were different from each other were mixed in a ratio specified below to give the desired mother liquid crystal A.

| | | |
|---|---|---|
| R⁹ = C₃H₇, | R¹⁰ = C₄H₉ | 27.6 wt% |
| R⁹ = C₄H₉, | R¹⁰ = C₂H₅ | 20.7 wt% |
| R⁹ = C₅H₁₁, | R¹⁰ = CH₃ | 20.7 wt% |
| R⁹ = C₃H₇, | R¹⁰ = C₂H₅ | 17.2 wt% |
| R⁹ = C₅H₁₁, | R¹⁰ = C₂H₅ | 13.8 wt% |
| | | 100.0 |

Δε= -1.5

### Composition of Mother Liquid Crystal B

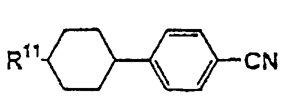

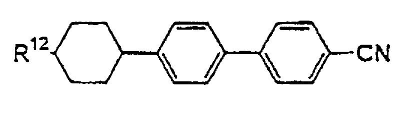

Four kinds of ester compounds represented by the foregoing general formulas and whose both terminal alkyl groups (R¹¹, R¹²) were different from each other were mixed in a ratio specified below to give the desired mother liquid crystal B.

| | |
|---|---|
| R¹¹ = C₃H₇ | 24.0 wt% |
| R¹¹ = C₅H₁₁ | 36.0 wt% |
| R¹¹ = C₇H₁₅ | 25.0 wt% |
| R¹² = C₅H₁₁ | 15.0 wt% |
| | 100.0 |

Δn = 0.137

### Example 1: Synthesis of 3-(4-(Trans-4-Propylcyclohexyl)Phenyl)-3,3-Difluoropropionic Acid 2,3-Difluoro-4-Ethoxyphenyl Ester (10)

### First Step: Synthesis of 3-(4-(Trans-4-Propylcyclohexyl)Phenyl)-3-Oxopropionic Acid (2)

A mixture of 4-(trans-4-propylcyclohexyl)bromobenzene (1) (281.0g, 1.00 mole), magnesium (29.17g, 1.20 mole) and THF (700 ml) was stirred under reflux for 6 hours, in a nitrogen gas atmosphere to give a Grignard reagent. After the completion of the reaction, the reaction solution was cooled to room temperature.

Malonic acid chloride (422.9g, 3.00 mole), iron(III) acetylacetone complex (17.66g, 0.0500 mole) and toluene (1400 ml) were mixed in another container and then cooled to -60 °C. To the mixture, there was dropwise added the Grignard reagent prepared above at -60 °C over 30 minutes and after the completion of the dropwise addition, they were reacted at that temperature for one hour. After the completion of the reaction, the temperature of the reaction solution was raised up to 0 °C, followed by dropwise addition of the reaction solution to a 1N dilute hydrochloric acid solution (1500 ml) under ice-cooling and extraction with ethyl acetate (5000 ml). The resulting organic phase was washed with water (1000 ml) and dried over anhydrous magnesium sulfate. The solvent was distilled off under reduced pressure, the resulting residue was purified through recrystallization (solvent: acetic acid) to give the title compound, 3-(4-(trans-4-propylcyclohexyl)phenyl)-3-oxopropionic acid ( 2)(109.5g, 0.380 mole; yield 38%). MS/288 (M ⁺ ).

### Second Step: Synthesis of Ethyl 3-(4-(Trans-4-Propylcyclohexyl)-Phenyl)-3-Oxopropionate (3)

There were mixed 3-(4-(trans-4-propylcyclohexyl)phenyl)-3-oxopropionic acid (2) (109.4g, 0.379 mole) prepared in the first step, sulfuric acid (1.86g, 0.0190 mole) and ethanol (87.53g, 1.90 mole) followed by reaction thereof under reflux for 8 hours. After the reaction, the reaction solution was cooled to room temperature and neutralized with a 1N sodium hydrogen carbonate aqueous solution (50 ml). The ethanol was distilled off under reduced pressure, followed by addition of water (1500 ml) to the resulting residue and extraction thereof with toluene (2000 ml). The resulting organic phase was washed with water (1000 ml) and dried over anhydrous magnesium sulfate. The solvent was distilled off under reduced pressure, the resulting residue was purified by silica gel chromatography (eluent: toluene) to give the title compound, ethyl 3-(4-(trans-4- propylcyclohexyl)phenyl)-3-oxopropionate(3) (98.10g, 0.310 mole; yield 82%). MS/316 (M ⁺).

### Third Step: Synthesis of Ethyl 3-(4-(Trans-4-Propylcyclohexyl)-Phenyl)-3,3-Difluoropropionate (4)

There were mixed ethyl 3-(4-(trans-4-propylcyclohexyl)phenyl)-3-oxopropionate (3) (98.01g, 0.310 mole) prepared in the second step and methylene chloride (400 ml), followed by cooling the mixture to -20 °C. Then a solution of DAST (99.85g, 0.619 mole) in methylene chloride (100 ml) was dropwise added to the mixture at that temperature. The mixture was reacted for 12 hours while gradually raising the temperature thereof up to room temperature. After the completion of the reaction, a 1N sodium hydrogen carbonate aqueous solution (500 ml) was added to the reaction solution, followed by extraction with methylene chloride (1000 ml). The resulting organic phase was washed with water (500 ml) and then dried over anhydrous magnesium sulfate. The solvent was distilled off under reduced pressure, the resulting residue was purified by silica gel chromatography (eluent: hexane) to give the title compound, ethyl 3-(4-(trans-4-propylcyclohexyl)phenyl)-3,3-difluoropropionate ( 4) (33.61g, 0.0993 mole; yield 32%). MS/338 (M ⁺ ).

### Fourth Step: Synthesis of 3-(4-(Trans-4-Propylcyclohexyl)Phenyl)-3,3-Difluoropropionic Acid (5)

To a mixture of sodium hydroxide (4.80g, 0.120 mole), water (100 ml) and methanol (100 ml), there was dropwise added ethyl 3-(4-(trans-4-propylcyclohexyl)phenyl)-3,3-difluoropropionate (4) (33.52g, 0.0990 mole) under ice-cooling. After the dropwise addition, the mixture was heated and reacted under reflux for 5 hours. After the completion of the reaction, the reaction solution was cooled and the methanol was distilled off under reduced pressure. Water (100 ml) was added to the resulting residue, followed by acidifying the same with a 1N dilute hydrochloric acid solution (150 ml) and extraction with ethyl acetate (300 ml). The resulting organic phase was washed with water (100 ml) and then dried over anhydrous magnesium sulfate. The solvent was distilled off under reduced pressure, the resulting residue was purified by recrystallization (solvent: acetic acid) to give the title compound, 3-(4-(trans-4-propylcyclohexyl)phenyl)-3,3-difluoropropionic acid (5) (28.86g, 0.0930 mole; yield 94%). MS/310 (M ⁺ ).

### Fifth Step: Synthesis of 2,3-Difluoro-4-Ethoxyphenyl Lithium (7)

To a solution of 2,3-difluoroethoxybenzene(6) (79.08g, 0.500 mole) in THF (300 ml), there was dropwise added, at -78°C, a solution of 1.56M sec-butyl lithium in hexane (385 ml, 0.600 mole) over 30 minutes, followed by stirring for 30 minutes to give a solution of the title compound, 2,3-difluoro-4-ethoxyphenyl lithium (7).

### Sixth Step: Synthesis of 2,3-Difluoro-4-Ethoxyphenyl Boric Acid (8)

The solution of 2,3-difluoro-4-ethoxyphenyl lithium (7) prepared in the fifth step was cooled to -78°C, a solution of trimethyl borate (62.35g, 0.600 mole) in THF (500 ml) was dropwise added thereto at that temperature, then the temperature of the reaction solution was gradually raised to room temperature, and the reaction was continued over 8 hours. After the completion of the reaction, water (500 ml) was added to the reaction solution under ice-cooling, then the reaction was ceased by the addition of a 1N dilute hydrochloric acid solution and the reaction solution was extracted with diethyl ether (1500 ml). The resulting organic phase was washed with water (500 ml) and then dried over anhydrous magnesium sulfate. The solvent was distilled off under reduced pressure to give the title compound, 2,3-difluoro-4-ethoxyphenyl boric acid (8) (59.62g, 0.295 mole; yield 59%). MS/202(M ⁺).

### Seventh Step: Synthesis of 2,3-Difluoro-4-Ethoxyphenol (9)

To a solution of 2,3-difluoro-4-ethoxyphenyl boric acid (8) (59.54g, 0.295 mole) prepared in the sixth step and formic acid (0.50g, 0.0295 mole) in ethanol (200 ml), there was dropwise added, under ice-cooling, 30% hydrogen peroxide (40.81g, 0.360 mole) and then they were reacted at 50°C for 2 hours. After the completion of the reaction, water (500 ml) was added to the reaction solution and then the latter was extracted with diethyl ether (500 ml). The resulting organic phase was washed with water (500 ml) and dried over anhydrous magnesium sulfate. The solvent was distilled off under reduced pressure and the resulting residue was purified by silica gel chromatography (eluent: 50:1 diethyl ether/methanol mixed solvent) to give the title compound, 2,3-difluoro-4-ethoxyphenol (9) (36.54g, 0.210 mole; yield 71%). MS/174(M ⁺ ).

### Eighth Step: Synthesis of 3-(4-(Trans-4-Propylcyclohexyl)Phenyl)-3,3-Difluoropropionic Acid 2,3-Difluoro-4-Ethoxyphenyl Ester (10)

There were dissolved, in methylene chloride (250 ml), 3-(4-(trans-4-propylcyclohexyl)phenyl)-3,3-difluoropropionic acid (5) (28.80g, 0. 0928 mole) prepared in the fourth step, 2,3-difluoro-4-ethoxyphenol (9) (17.78g, 0.102 mole) prepared in the seventh step and DMAP (2.27g, 0.0186 mole). After addition of DCC (22.97g, 0.111 mole) under ice-cooling, the mixture was reacted at room temperature for 12 hours. After the completion of the reaction, the reaction solution was filtered, the solvent was distilled off from the filtrate under reduced pressure, followed by purification of the resulting residue through silica gel column chromatography (eluent: hexane) and then recrystallization (hexane-toluene) to give 3-(4-(trans-4-propylcyclohexyl)phenyl)-3,3-difluoropropionic acid 2,3-difluoro-4-ethoxyphenyl ester (10) (28.03g, 0.0601 mole; yield 65%).

MS/467(M ⁺); Δε= -6.8; Δn = 0.137.

The following compounds can be prepared according to the method described in Example 1.

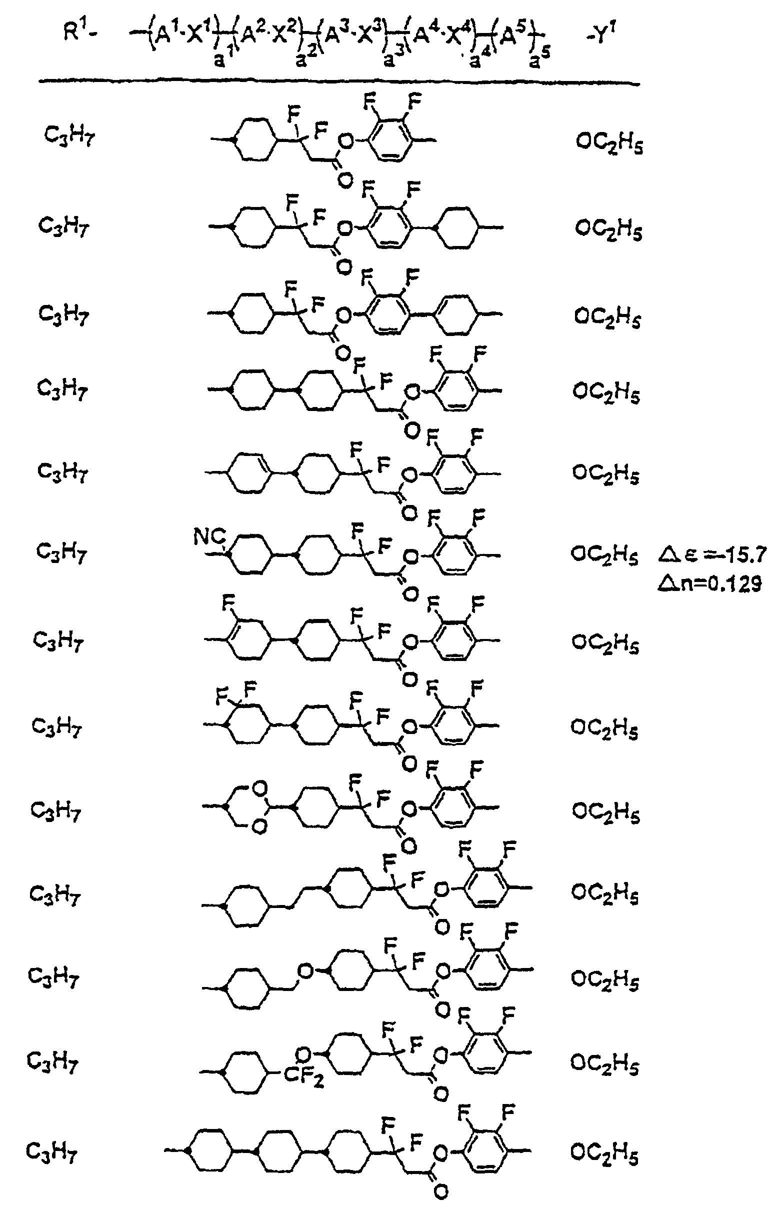

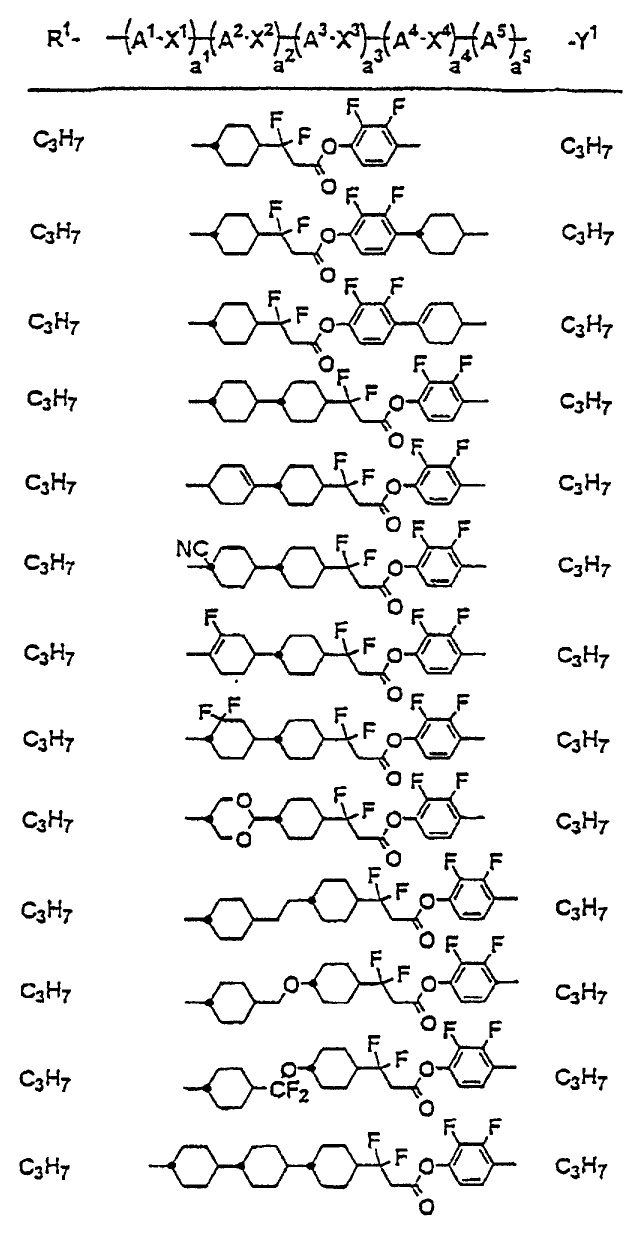

As has been described above in detail, the present invention can provide a liquid crystal compound which has a negative dielectric anisotropy value whose absolute value is large, a low viscosity, a controlled optical anisotropy value, a high specific resistance and a high voltage-holding ratio and which is stable against heat and irradiation with ultraviolet rays as well as a liquid crystal composition containing the compound and a liquid crystal display device produced using the composition.

## Claims

1. A liquid crystal compound represented by the following general formula (3):
R¹-(A¹-X¹)ₐ₁-(A²-X²)ₐ₂-(A³-X³)ₐ₃-(A⁴-X⁴)ₐ₄-(A⁵) ₐ₅-Y¹ (3)
wherein R¹ and Y¹ each independently represents an alkyl group having 1 to 20 carbon atoms, a hydrogen atom, a halogen atom or a cyano group, provided that at least one of the methylene groups present in the alkyl group, which are not adjacent to one another may be substituted with an oxygen, sulfur or nitrogen atom, -C≡C-, a silylene group wherein at least one hydrogen atom may be substituted with an alkyl group having 1 to 10 carbon atoms, or a vinylene group, or that at least one hydrogen atom of the alkyl group may be substituted with a fluorine or chlorine atom, or that these alkyl groups may be optically active ones; X¹, X², X³ and X⁴ each independently represents a single bond, -(CH₂)₂-, -CH=CH-, -C≡C-, -COO-, -OCO-, -CH₂O-, -OCH₂-, -(CH₂)₄-, -(CH₂)₃O-, -O (CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC≡C-, -C≡CCH=CH-, or a group represented by the general formula (1) 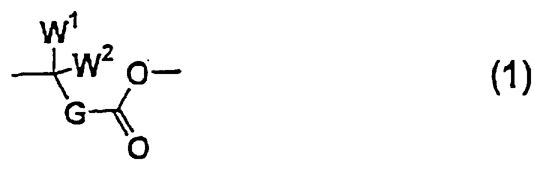 (wherein G represents an alkylene group having 1 to 3 carbon atoms wherein at least one hydrogen atom constituting the alkylene group may be substituted with a fluorine or chlorine atom; W¹ and W² each independently represents a hydrogen, fluorine or chlorine atom, provided that at least one of W¹ and W² represent a fluorine or chlorine atom), provided that at least one of the substituents X¹, X², X³ and X⁴ represent a group represented by the foregoing general formula (1); the rings A¹, A², A³, A⁴ and A⁵ each independently represents a trans-cyclohexane-1,4-diyl, cyclohexane-1-en-1,4-diyl, 1,4-phenylene, bicyclo[1. 1.1]pentane-1,3-diyl group, or a ring represented by the general formula (2) 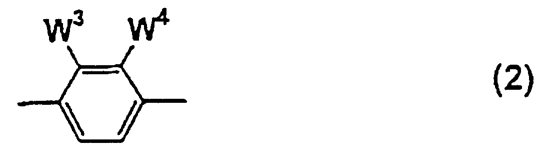 wherein W³ and W⁴ each independently represents a fluorine or chlorine atom), provided that at least one of these rings A¹, A², A³, A⁴ and A⁵ represent a ring represented by Formula (2), that the carbon atoms constituting these rings may be substituted with a nitrogen, oxygen or sulfur atom and that each hydrogen atom present on the rings may be substituted with a halogen atom or a cyano group; a¹, a², a³, a⁴ and a⁵ each independently represents 0 or 1, provided that they satisfy the relation: 4 ≥ a¹+a²+a³+a⁴+a⁵ ≧ 2. .

2. The liquid crystal compound of claim 1 wherein the group represented by the general formula (1) is -CF₂CH₂COO- and that represented by the general formula (2) is 2,3-difluorobenzene-1,4-diyl.

3. A liquid crystal composition comprising at least two components including at least one liquid crystal compound as set forth in any one of claims 1 or 2.

4. A liquid crystal composition comprising at least one liquid crystal compound as set forth in any one of claims 1 or 2 as a first component and at least one compound selected from the group consisting of those represented by the following general formulas (4), (5) and (6): 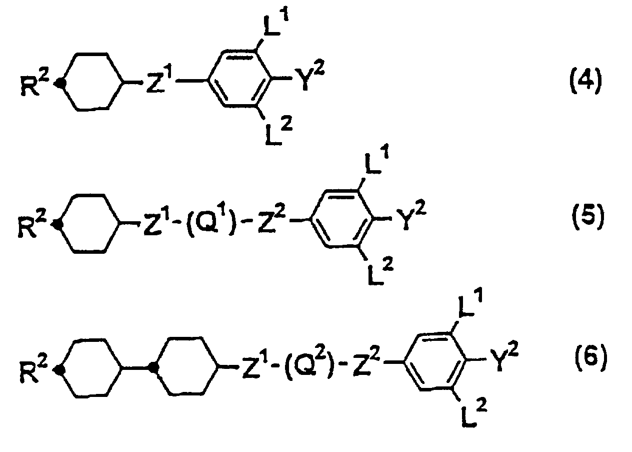 (wherein R² represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y² represents a fluorine or chlorine atom, -OCF₃, -OCF₂H, -CF₃, -CF₂H, -CFH₂, -OCF₂CF₂H or -OCF₂CFHCF₃; L¹ and L² each independently represents a hydrogen or fluorine atom; Z¹ and Z² each independently represents -(CH₂)₂-, -(CH₂)₄-, -COO-, -CF₂O-, -OCF₂-, -CH=CH- or a single bond; the ring Q¹ represents a trans-cyclohexane-1,4-diyl or 1,3-dioxane-2,5-diyl group, or a 1,4-phenylene group whose hydrogen atom may be substituted with a fluorine atom; the ring Q² represents a trans-cyclohexane-1,4-diyl group, or a 1,4-phenylene group whose hydrogen atom may be substituted with a fluorine atom; and each atom constituting the compound may be substituted with its isotope) as a second component.

5. A liquid crystal composition comprising at least one liquid crystal compound as set forth in any one of claims 1 or 2 as a first component and at least one compound selected from the group consisting of those represented by the following general formulas (7) and (8) as a second component: 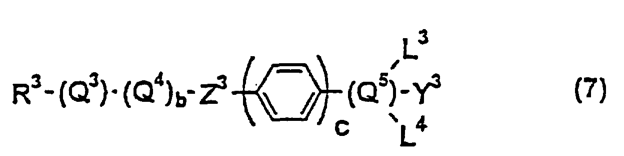 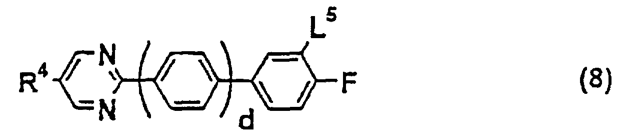 wherein R³ and R⁴ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y³ represents a cyano group or -C ≡C-CN; the ring Q³ represents a trans-cyclohexane-1,4-diyl, 1,4-phenylene, 1,3-dioxane-2,5-diyl or pyrimidine-2,5-diyl group; the ring Q⁴ represents a trans-cyclohexane-1,4-diyl or pyrimidine-2,5-diyl group, or a 1,4-phenylene group whose hydrogen atom may be substituted with a fluorine atom; the ring Q⁵ represents a trans-cyclohexane-1,4-diyl or 1,4-phenylene group; Z³ represents -(CH₂)₂-, -COO- or a single bond; L³, L⁴ and L⁵ each independently represents a hydrogen or fluorine atom; b, c and d each independently represents 0 or 1; and each atom constituting the compound may be substituted with its isotope.

6. A liquid crystal composition comprising at least one liquid crystal compound as set forth in any one of claim 1 or 2 as a first component; at least one compound selected from the group consisting of those represented by the general formulas (4), (5) and (6) as defined in claim 4 as a second component; and at least one compound selected from the group consisting of those represented by the following general formulas (9), (10) and (11) as a third component:
R⁵-(Q⁶)-Z⁴-(Q⁷)-Z⁵-R⁶ (9)
R⁵-(Q⁶)-Z⁴-(Q⁷)-Z⁵-(Q⁸)-R⁶ 10) 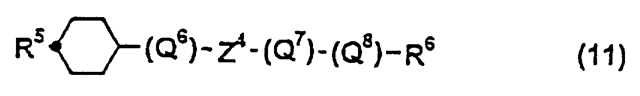
wherein R⁵ and R⁶ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁶, Q⁷ and Q⁸ each independently represents a trans-cyclohexane-1,4-diyl or pyrimidine-2,5-diyl group, or a 1, 4-phenylene group whose hydrogen atom may be substituted with a fluorine atom; Z⁴ and Z⁵ each independently represents -C≡C-, -COO-, -(CH ₂)₂-, -CH=CH- or a single bond; and each atom constituting the compound may be substituted with its isotope.

7. A liquid crystal composition comprising at least one liquid crystal compound as set forth in any one of claims 1 or 2 as a first component and at least one compound selected from the group consisting of those represented by the following general formulas (12), (13) and (14) as a second component: 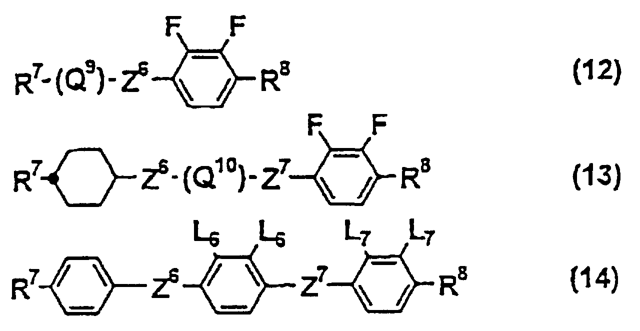 wherein R⁷ and R⁸ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁹ and Q¹⁰ each independently represents a trans-cyclohexane-1,4-diyl or 1,4-phenylene group; L⁶ and L⁷ each independently represents a hydrogen or fluorine atom, provided that L⁶ and L⁷ do not simultaneously represent a hydrogen atom; Z⁶ and Z⁷ each independently represents -(CH₂)₂-, -COO- or a single bond; and each atom constituting the compound may be substituted with its isotope.

8. A liquid crystal composition comprising at least one liquid crystal compound as set forth in any one of claims 1 or 2 as a first component; at least one compound selected from the group consisting of those represented by the general formulas (9), (10) and (11) as defined in claim 6 as a second component; and at least one compound selected from the group consisting of those represented by the general formulas (12), (13) and 14) as defined in claim 7 as a third component.

9. A liquid crystal composition comprising at least one liquid crystal compound as set forth in any one of claims 1 or 2 as a first component; at least one compound selected from the group consisting of those represented by the general formulas (7) and (8) as defined in claim 5 as a second component; and at least one compound selected from the group consisting of those represented by the general formulas (9), (10) and (11) as defined in claim 6 as a third component.

10. A liquid crystal composition comprising at least one liquid crystal compound as set forth in any one of claims 1 or 2 as a first component; at least one compound selected from the group consisting of those represented by the general formulas (4), (5) and (6) as defined in claim 4 as a second component; at least one compound selected from the group consisting of those represented by the general formulas (7) and (8) as defined in claim 5 as a third component; and at least one compound selected from the group consisting of those represented by the general formulas (9), (10) and (11) as defined in claim 6 as a fourth component.

11. A liquid crystal composition comprising at least one optically active compound in addition to a liquid crystal composition as set. forth in any one of claims 3 to 10.

12. A liquid crystal display device constituted by the use of a liquid crystal composition as set forth in any one of claims 3 to 11.

## Patentansprüche

1. Flüssigkristallverbindung, dargestellt durch die folgende allgemeine Formel (3):
R¹-(A¹-X¹)ₐ₁-(A²-X²)ₐ₂-(A³-X³)ₐ₃-(A⁴-X⁴)ₐ₄-(A⁵)ₐ₅-Y¹ (3)
wobei R¹ und Y¹ jeweils unabhängig voneinander eine Alkylgruppe mit 1 bis 20 Kohlenstoffatomen, ein Wasserstoffatom, ein Halogenatom oder eine Cyanogruppe darstellen, vorausgesetzt, daß mindestens eine der Methylengruppen, die in der Alkylgruppe vorhanden sind, die nicht benachbart sind, mit einem Sauerstoff-, einem Schwefel- oder einem Stickstoffatom, einer -C≡C-Gruppe, einer Silylengruppe, wobei mindestens ein Wasserstoffatom mit einer Alkylgruppe mit 1 bis 10 Kohlenstoffatomen substituiert sein kann, oder einer Vinylengruppe substituiert sein kann, oder daß mindestens ein Wasserstoffatom der Alkylgruppe mit einem Fluor- oder einem Chloratom substituiert sein kann, oder daß diese Alkylgruppen optisch aktive Gruppen sein können; wobei X¹, X², X³ und X⁴ jeweils unabhängig voneinander eine Einfachbindung, -(CH₂)₂-, -CH=CH-, -C≡C-, -COO-, -OCO-, -CH₂O-, OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC≡C-, -C≡CCH=CH- oder eine Gruppe, dargestellt durch die allgemeine Formel (1): 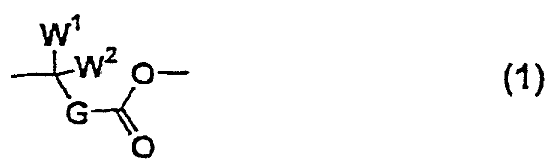 (wobei G eine Alkylengruppe mit 1 bis 3 Kohlenstoffatomen darstellt, worin mindestens ein Wasserstoffatom, welches die Alkylengruppe aufbaut, mit einem Fluor- oder einem Chloratom substituiert sein kann; W¹ und W² jeweils unabhängig voneinander ein Wasserstoff-, ein Fluor- oder ein Chloratom darstellen, vorausgesetzt, daß mindestens eine Gruppe W¹ und W² ein Fluor- oder ein Chloratom darstellt), vorausgesetzt, daß mindestens einer der Substituenten X¹, X², X³ und X⁴ eine Gruppe darstellt, die durch die vorhergehende allgemeine Formel (1) dargestellt ist; die Ringe A¹, A², A³, A⁴ und A⁵ jeweils unabhängig voneinander eine trans-Cyclohexan-1,4-diyl-, eine Cyclohexan-1-en-1,4-diyl-, eine 1,4-Phenylen-, eine Bicyclo[1.1.1]pentan-1,3-diylgruppe oder einen Ring, dargestellt durch die allgemeine Formel (2): 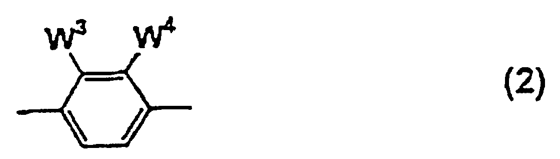 darstellen,
(wobei W³ und W⁴ jeweils unabhängig voneinander ein Fluor- oder ein Chloratom darstellen), vorausgesetzt, daß mindestens einer dieser Ringe A¹, A², A³, A⁴ und A⁵ einen Ring darstellt, der durch die Formel (2) dargestellt ist, daß die Kohlenstoffatome, die diesen Ring aufbauen, mit einem Stickstoff-, einem Sauerstoff- oder einem Schwefelatom substituiert sein können, und daß jedes Wasserstoffatom, welches an diesen Ringen vorhanden ist, mit einem Halogenatom oder einer Cyanogruppe substituiert sein kann; a¹, a², a³, a⁴ und a⁵ jeweils unabhängig voneinander 0 oder 1 darstellen, vorausgesetzt, daß sie die Beziehung: 4 ≥ a¹+a²+a³+a⁴+a⁵ ≥ 2 erfüllen.

2. Flüssigkristallverbindung nach Anspruch 1, wobei die durch die allgemeine Formel (1) dargestellte Gruppe eine -CF₂CH₂COO-Gruppe ist und jene, durch die allgemeine Formel (2) dargestellte Gruppe, eine 2,3-Difluorbenzol-1,4-diylgruppe ist.

3. Flüssigkristallzusammensetzung, umfassend mindestens zwei Komponenten, die mindestens eine Flüssigkristallverbindung nach einem der Ansprüche 1 oder 2 einschließen.

4. Flüssigkristallzusammensetzung, umfassend mindestens eine Flüssigkristallverbindung nach Anspruch 1 oder 2 als eine erste Komponente und mindestens eine Verbindung, ausgewählt aus der Gruppe, bestehend aus solchen Verbindungen, die durch die folgenden allgemeinen Formeln (4), (5) und (6) dargestellt sind: 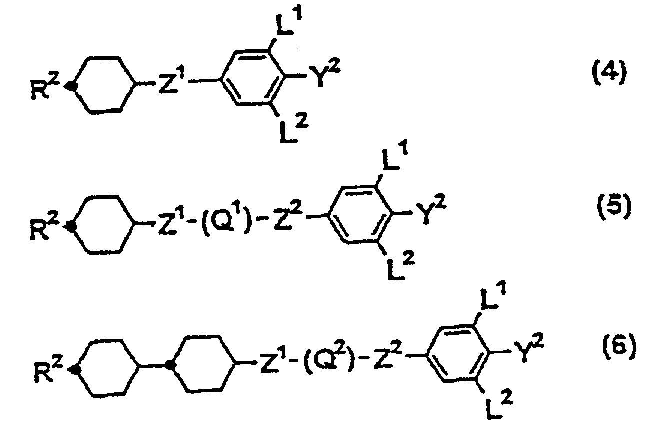 (wobei R² eine Alkylgruppe mit 1 bis 10 Kohlenstoffatomen darstellt, vorausgesetzt, daß mindestens eine der Methylengruppen, die in der Alkylgruppe vorhanden sind, die nicht benachbart sind, mit einem Sauerstoffatom oder einer -CH=CH-Gruppe substituiert sein kann, und daß mindestens eines der Wasserstoffatome, die in der Alkylgruppe vorhanden sind, mit einem Fluoratom substituiert sein kann; Y² ein Fluor- oder ein Chloratom, -OCF₃, -OCF₂H, -CF₃, -CF₂H, -CFH₂, -OCF₂CF₂H oder -OCF₂CFHCF₃ darstellt; L¹ und L² jeweils unabhängig voneinander ein Wasserstoff- oder ein Fluoratom darstellen; Z¹ und Z² jeweils unabhängig voneinander -(CH₂)₂-, -(CH₂)₄-, -COO-, -CF₂O-, -OCF₂-, -CH=CH- oder eine Einfachbindung darstellen; der Ring Q¹ eine trans-Cyclohexan-1,4-diyloder eine 1,3-Dioxan-2,5-diylgruppe oder eine 1,4-Phenylengruppe, deren Wasserstoffatom(e) mit einem Fluoratom substituiert sein kann, darstellt, der Ring Q² eine trans-Cyclohexan-1,4-diylgruppe oder eine 1,4-Phenylengruppe, deren Wasserstoffatom(e) mit einem Fluoratom substituiert sein kann, darstellt; und jedes Atom, welches die Verbindung aufbaut, mit dessen Isotop substituiert sein kann) als eine zweite Komponente.

5. Flüssigkristallzusammensetzung, umfassend mindestens eine Flüssigkristallverbindung nach Anspruch 1 oder 2 als eine erste Komponente und mindestens eine Verbindung, ausgewählt aus der Gruppe, bestehend aus solchen, die durch die folgenden allgemeinen Formeln (7) und (8) dargestellt sind, als eine zweite Komponente: 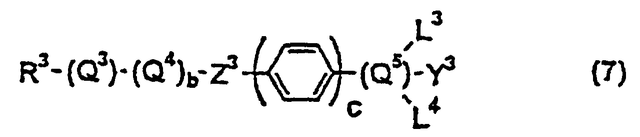 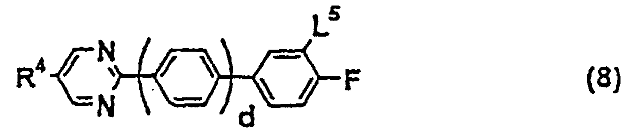 wobei R³ und R⁴ jeweils unabhängig voneinander eine Alkylgruppe mit 1 bis 10 Kohlenstoffatomen darstellen, vorausgesetzt, daß mindestens eine der Methylengruppen, die in der Alkylgruppe vorhanden sind, welche nicht benachbart sind, mit einem Sauerstoffatom oder einer -CH=CH-Gruppe substituiert sein kann, und daß mindestens eines der Wasserstoffatome, die in der Alkylgruppe vorhanden sind, mit einem Fluoratom substituiert sein kann; Y³ eine Cyanogruppe oder eine Gruppe -C≡C-CN darstellt; der Ring Q³ eine trans-Cyclohexan-1,4-diyl-, eine 1,4-Phenylen-, eine 1,3-Dioxan-2,5-diyl- oder eine Pyrimidin-2,5-diylgruppe darstellt; der Ring Q⁴ eine trans-Cyclohexan-1,4-diyl- oder eine Pyrimidin-2,5-diylgruppe oder eine 1,4-Phenylengruppe, deren Wasserstoffatom(e) mit einem Fluoratom substituiert sein kann, darstellt; der Ring Q⁵ eine trans-Cyclohexan-1,4-diyl- oder eine 1,4-Phenylengruppe darstellt; Z³ -(CH₂)₂-, -COO- oder eine Einfachbindung darstellt; L³, L⁴ und L⁵ jeweils unabhängig voneinander ein Wasserstoff- oder ein Fluoratom darstellen; b, c und d jeweils unabhängig voneinander 0 oder 1 darstellen; und jedes Atom, welches die Verbindung aufbaut, mit dessen Isotop substituiert sein kann.

6. Flüssigkristallzusammensetzung, umfassend mindestens eine Flüssigkristallverbindung nach Anspruch 1 oder 2 als eine erste Komponente, mindestens eine Verbindung, ausgewählt aus der Gruppe, bestehend aus solchen, die durch die allgemeinen Formeln (4), (5) und (6), wie in Anspruch 4 definiert, dargestellt sind, als eine zweite Komponente und mindestens eine Verbindung, ausgewählt aus der Gruppe, bestehend aus solchen, die durch die folgenden allgemeinen Formeln (9), (10) und (11) dargestellt sind, als eine dritte Komponente:
R⁵-(Q⁶)-Z⁴-(Q⁷)-Z⁵-R⁶ (9)
R⁵-(Q⁶)-Z⁴-(Q⁷)-Z⁵-(Q⁸)-R⁶ (10) 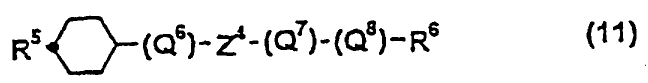
wobei R⁵ und R⁶ jeweils unabhängig voneinander eine Alkylgruppe mit 1 bis 10 Kohlenstoffatomen darstellen, vorausgesetzt, daß mindestens eine der Methylengruppen, die in der Alkylgruppe vorhanden sind, welche nicht benachbart sind, mit einem Sauerstoffatom oder einer -CH=CH-Gruppe substituiert sein kann, und daß mindestens eines der Wasserstoffatome, die in der Alkylgruppe vorhanden sind, mit einem Fluoratom substituiert sein kann; die Ringe Q⁶, Q⁷ und Q⁸ jeweils unabhängig voneinander eine trans-Cyclohexan-1,4-diyl- oder eine Pyrimidin-2,5-diylgruppe oder eine 1,4-Phenylengruppe, deren Wasserstoffatom(e) mit einem Fluoratom substituiert sein kann, darstellen; Z⁴ und Z⁵ jeweils unabhängig voneinander -C≡C-, -COO-, -(CH₂)₂-, -CH=CH- oder eine Einfachbindung darstellen; und jedes Atom, welches die Verbindung aufbaut, mit dessen lsotop substituiert sein kann.

7. Flüssigkristallzusammensetzung, umfassend mindestens eine Flüssigkristallverbindung nach Anspruch 1 oder 2 als eine erste Komponente und mindestens eine Verbindung, ausgewählt aus der Gruppe, bestehend aus solchen, die durch die folgenden allgemeinen Formeln (12), (13) und (14) dargestellt sind, als eine zweite Komponente: 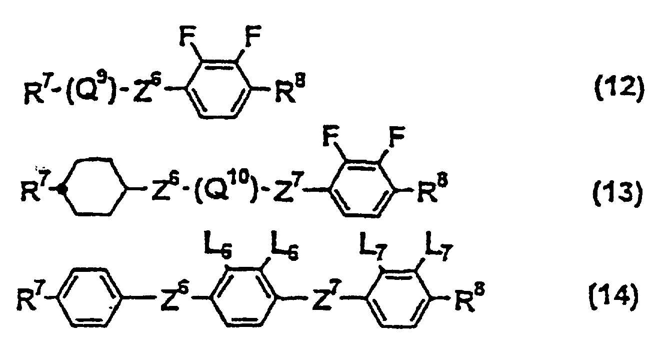 wobei R⁷ und R⁸ jeweils unabhängig voneinander eine Alkylgruppe mit 1 bis 10 Kohlenstoffatomen darstellen, vorausgesetzt, daß mindestens eine der Methylengruppen, die in der Alkylgruppe vorhanden sind, welche nicht benachbart sind, mit einem Sauerstoffatom oder einer -CH=CH-Gruppe substituiert sein kann, und daß mindestens eines der Wasserstoffatome, die in der Alkylgruppe vorhanden sind, mit einem Fluoratom substituiert sein kann; die Ringe Q⁹ und Q¹⁰ jeweils unabhängig voneinander eine trans-Cyclohexan-1,4-diyl- oder eine 1,4-Phenylengruppe darstellen; L⁶ und L⁷ jeweils unabhängig voneinander ein Wasserstoff- oder ein Fluoratom darstellen, vorausgesetzt, daß L⁶ und L⁷ nicht gleichzeitig ein Wasserstoffatom darstellen; Z⁶ und Z⁷ jeweils unabhängig voneinander -(CH₂)₂-, -COO- oder eine Einfachbindung darstellen; und jedes Atom, welches die Verbindung aufbaut, mit dessen Isotop substituiert sein kann.

8. Flüssigkristallzusammensetzung, umfassend mindestens eine Flüssigkristallverbindung nach Anspruch 1 oder 2 als eine erste Komponente, mindestens eine Verbindung, ausgewählt aus der Gruppe, bestehend aus solchen, die durch die allgemeinen Formeln (9), (10) und (11), wie in Anspruch 6 definiert, dargestellt sind, als eine zweite Komponente und mindestens eine Komponente, ausgewählt aus der Gruppe, bestehend aus solchen, die durch die allgemeinen Formeln (12), (13) und (14), wie in Anspruch 7 definiert, dargestellt sind, als eine dritte Komponente.

9. Flüssigkristallzusammensetzung, umfassend mindestens eine Flüssigkristallverbindung nach Anspruch 1 oder 2 als eine erste Komponente, mindestens eine Verbindung, ausgewählt aus der Gruppe, bestehend aus solchen, die durch die allgemeinen Formeln (7) und (8), wie in Anspruch 5 definiert, dargestellt sind, als eine zweite Komponente und mindestens eine Verbindung, ausgewählt aus der Gruppe, bestehend aus solchen, die durch die allgemeinen Formeln (9), (10) und (11), wie in Anspruch 6 definiert, dargestellt sind, als eine dritte Komponente.

10. Flüssigkristallzusammensetzung, umfassend mindestens eine Flüssigkristallverbindung nach Anspruch 1 oder 2 als eine erste Komponente, mindestens eine Verbindung, ausgewählt aus die Gruppe, bestehend aus solchen, die durch die allgemeinen Formeln (4), (5) und (6), wie in Anspruch 4 definiert, dargestellt sind, als eine zweite Komponente, mindestens eine Verbindung, ausgewählt aus der Gruppe, bestehend aus solchen, die durch die allgemeinen Formeln (7) und (8), wie in Anspruch 5 definiert, dargestellt sind, als eine dritte Komponente und mindestens eine Verbindung, ausgewählt aus der Gruppe, bestehend aus solchen, die durch die allgemeinen Formeln (9), (10) und (11), wie in Anspruch 6 definiert, dargestellt sind, als eine vierte Komponente.

11. Flüssigkristallzusammensetzung, umfassend mindestens eine optisch aktive Verbindung zusätzlich zu einer Flüssigkristallzusammensetzung nach einem der Ansprüche 3 bis 10.

12. Flüssigkristallanzeigevorrichtung, die unter Verwendung einer Flüssigkristallzusammensetzung nach einem der Ansprüche 3 bis 11 aufgebaut ist.

## Revendications

1. Composé du type cristal liquide représenté par la formule générale (3) suivante :
R¹-(A¹-X¹)ₐ₁-(A²-X²)ₐ₂-(A³-X³)ₐ₃-(A⁴-X⁴)ₐ₄-(A⁵) ₐ₅-Y¹ (3)
dans laquelle R¹ et Y¹ représentent chacun, indépendamment, un groupe alkyle ayant 1 à 20 atomes de carbone, un atome d'hydrogène, un atome d'halogène ou un groupe cyano, sous réserve qu'au moins un des groupes méthylène présents dans le groupe alkyle, qui ne sont pas adjacents les uns aux autres, soit substitué avec un atome d'oxygène, de soufre ou d'azote, un groupe -C≡C-, un groupe sylilène dans lequel au moins un atome d'hydrogène peut être substitué avec un groupe alkyle ayant 1 à 10 atomes de carbone, ou un groupe vinylène, ou qu'au moins un atome d'hydrogène du groupe alkyle soit substitué avec un atome de fluor ou de chlore, ou bien que ces groupes alkyle puissent être optiquement actifs ; X¹, X², X³ et X⁴ représentent chacun, indépendamment, une liaison simple, un groupe -(CH₂)₂-, -CH=CH-, -C≡C-, -COO-, -OCO-, -CH₂O-, -OCH2-, -(CH₂)₄, - (CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, - (CH₂)₂COO-, -OCO(CH₂)₂-, -CH=CHCOO-, -OCOCH=CH-, -CH=CHC≡C-, -C≡CCH=CH-, ou un groupe représenté par la formule générale (1) 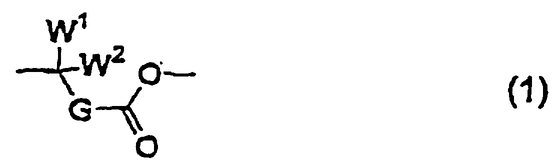 (dans laquelle G représente un groupe alkylène ayant 1 à 3 atomes de carbone dans lequel au moins un atome d'hydrogène présent dans le groupe alkylène peut être substitué avec un atome de fluor ou de chlore ; W¹ et W² représentent chacun, indépendamment, un atome d'hydrogène, de fluor ou de chlore, sous réserve qu'au moins un des groupes W¹ et W² représentent chacun, indépendamment, un atome d'hydrogène, de fluor ou de chlore, sous réserve qu'au moins un des groupes W¹ et W² représente un atome de fluor ou de chlore), sous réserve qu'au moins un des substituants X¹, X², X³, X⁴ représente un groupe représenté par la formule générale précitée ; les noyaux A¹, A², A³, A⁴ et A⁵ représentent chacun, indépendamment, un groupe trans-cyclohexane-1,4-diyle, cyclohexane-l-ène-1,4-diyle, 1,4-phénylène ou bicyclo[1.1.1]pentane-1,3-diyle, ou un noyau représenté par la formule générale (2) 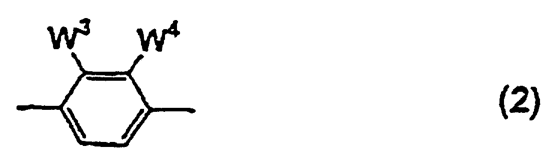 (dans laquelle W³ et W⁴ représentent chacun, indépendamment, un atome de fluor ou de chlore), sous réserve qu'au moins un de ces noyaux A¹, A², A³, A⁴ et A⁵ représente un noyau représenté par la formule (2), que les atomes de carbone constituant ces noyaux soient substitués avec un atome d'azote, d'oxygène ou de soufre et que chaque atome d'hydrogène présent sur les noyaux puisse être substitué avec un atome d'halogène ou un groupe cyano ; A¹, A², A³, A⁴ et A⁵ sont chacun égaux, indépendamment, à 0 ou 1, sous réserve qu'ils satisfassent la relation 4 ≥ a¹+a²+a³+a⁴+a⁵ ≥ 2.

2. Composé du type cristal liquide suivant la revendication 1, dans lequel le groupe représenté par la formule générale (1) est un groupe -CF₂CH₂COO- et celui représenté par la formule générale (2) est un groupe 2,3-difluorobenzène-1,4-diyle.

3. Composition de cristal liquide comprenant au moins deux constituants comprenant au moins un composé du type cristal liquide répondant à la définition suivant l'une quelconque des revendications 1 et 2.

4. Composition de cristal liquide comprenant au moins un composé du type cristal liquide répondant à la définition suivant l'une quelconque des revendications 1 et 2 comme premier constituant et au moins un composé choisi dans le groupe consistant en ceux représentés par les formules générales (4), (5) et (6) suivantes : 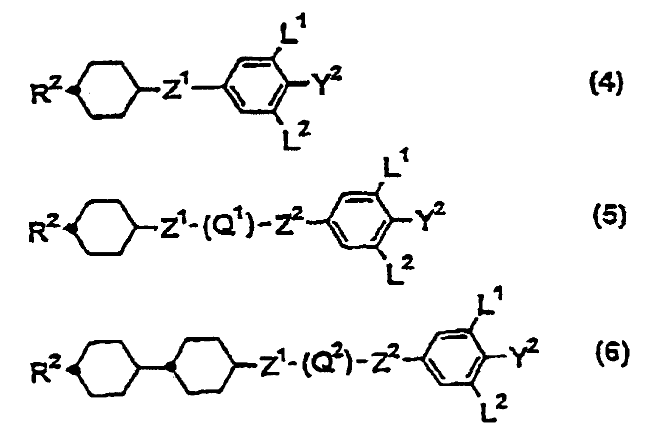 (dans lesquelles R² représente un groupe alkyle ayant 1 à 10 atomes de carbone, sous réserve qu'au moins un des groupes méthylène présents dans le groupe alkyle, qui ne sont pas adjacents les uns aux autres, soit substitué avec un atome d'oxygène ou un groupe -CH=CH-, et qu'au moins un des atomes d'hydrogène présents dans le groupe alkyle soit substitué avec un atome de fluor ; Y² représente un atome de fluor ou de chlore, un groupe -OCF₃, -OCF₂H, -CF₃, -CF₂H, -CFH₂, -OCF₂CF₂H ou -OCF₂CFHCF₃ ; L¹ et L² représentent chacun, indépendamment, un atome d'hydrogène ou de fluor ; Z¹ et Z² représentent chacun, indépendamment, un groupe -(CH₂)₂-, -(CH₂)₄-, -COO-, -CF₂O-, -OCF₂-, -CH=CH- ou une liaison simple ; le noyau Q¹ représente un groupe trans-cyclohexane-1,4-diyle ou 1,3-dioxanne-2,5-diyle, ou un groupe 1,4-phénylène dont un atome d'hydrogène est substitué avec un atome de fluor ; le noyau Q² représente un groupe trans-cyclohexane-1,4-diyle, ou un groupe 1,4-phénylène dont un atome d'hydrogène est substitué avec un atome de fluor ; et chaque atome présent dans le composé est substitué par son isotope) comme second constituant.

5. Composition de cristal liquide comprenant au moins un composé du type cristal liquide répondant à la définition suivant l'une quelconque des revendications 1 et 2 comme premier constituant et au moins un composé choisi dans le groupe consistant en ceux représentés par les formules générales (7) et (8) suivantes comme second constituant : 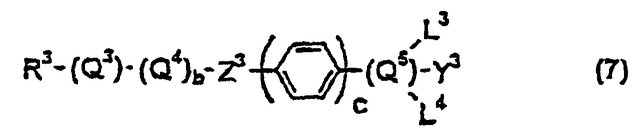 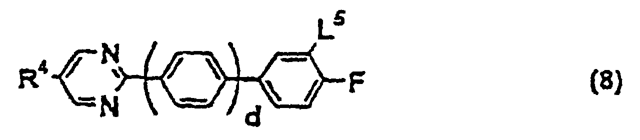 formules dans lesquelles R³ et R⁴ représentent chacun, indépendamment, un groupe alkyle ayant 1 à 10 atomes de carbone, sous réserve qu'au moins un des groupes méthylène présents dans le groupe alkyle, qui ne sont pas adjacents les uns aux autres, soit substitué avec un atome d'oxygène ou un groupe -CH=CH- et qu'au moins un des atomes d'hydrogène présents dans le groupe alkyle soit substitué avec un atome de fluor ; Y³ représente un groupe cyano ou un groupe -C≡C-CN ; le noyau Q³ représente un groupe trans-cyclohexane-1,4-diyle, 1,4-phénylène, 1,3-dioxanne-2,5-diyle ou pyrimidine-2,5-diyle ; le noyau Q⁴ représente un groupe trans-cyclohexane-1,4-diyle ou pyrimidine-2,5-diyle, ou un groupe 1,4-phénylène dont un atome d'hydrogène est substitué avec un atome de fluor ; le noyau Q⁵ représente un groupe trans-cyclohexane-1,4-diyle ou 1,4-phénylène ; Z³ représente un groupe -(CH₂)₂-, -COO- ou une liaison simple ; L³, L⁴ et L⁵ représentent chacun, indépendamment, un atome d'hydrogène ou de fluor ; b, c et d sont chacun égaux, indépendamment, à 0 ou 1 ; et chaque atome présent dans le composé est substitué par son isotope.

6. Composition de cristal liquide comprenant au moins un composé du type cristal liquide répondant à la définition suivant l'une quelconque des revendications 1 et 2 comme premier constituant ; au moins un composé choisi dans le groupe consistant en les composés représentés par les formules générales (4), (5) et (6) définis dans la revendication 4 comme second constituant ; et au moins un composé choisi dans le groupe consistant en les composés représentés par les formules générales (9), (10) et (11) suivantes comme troisième constituant : 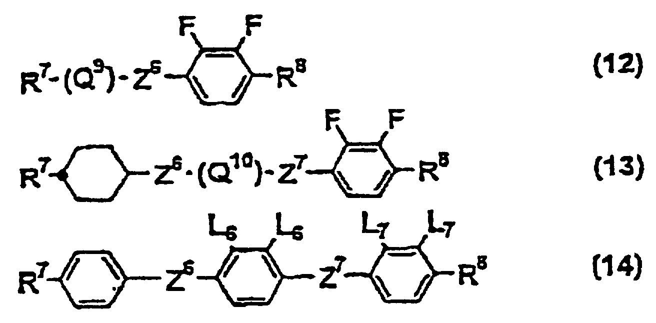 formules dans lesquelles R⁵ et R⁶ représentent chacun, indépendamment, un groupe alkyle ayant 1 à 10 atomes de carbone, sous réserve qu'au moins un des groupes méthylène présents dans le groupe alkyle, qui ne sont pas adjacents les uns aux autres, soit substitué avec un atome d'oxygène ou un groupe -CH=CH-, et qu'au moins un des atomes d'hydrogène présents dans le groupe alkyle soit substitué avec un atome de fluor ; les noyaux Q⁶, Q⁷ et Q⁸ représentent chacun, indépendamment, un groupe trans-cyclohexane-1,4-diyle ou pyrimidine-2,5-diyle, ou un groupe 1,4-phénylène dont un atome d'hydrogène peut être substitué avec un atome de fluor ; Z⁴ et Z⁵ représentent chacun, indépendamment, un groupe -C≡C-, -COO-, -(CH₂)₂-, -CH=CH- ou une liaison simple ; et chaque atome présent dans le composé est substitué par son isotope.

7. Composition de cristal liquide comprenant au moins un composé du type cristal liquide répondant à la définition suivant l'une quelconque des revendications 1 et 2 comme premier constituant, et au moins un composé choisi dans le groupe consistant en les composés représentés par les formules générales (12), (13) et (14) suivantes comme second constituant :
R⁵-(Q⁶)-Z⁴-(Q⁷)-Z⁵-R⁶ (9) (9)
R⁵-(Q⁶)-Z⁴-(Q⁷)-Z⁵-(Q⁸)-R⁶ (10) 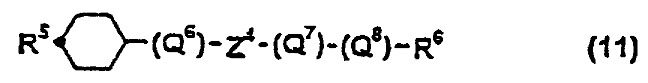
formules dans lesquelles R⁷ et R⁸ représentent chacun, indépendamment, un groupe alkyle ayant 1 à 10 atomes de carbone, sous réserve qu'au moins un des groupes méthylène présents dans le groupe alkyle, qui ne sont pas adjacents les uns aux autres, soit substitué avec un atome d'oxygène ou un groupe -CH=CH- et qu'au moins un des atomes d'hydrogène présents dans le groupe alkyle soit substitué avec un atome de fluor ; les noyaux Q⁹ et Q¹⁰ représentent chacun, indépendamment, un groupe trans-cyclohexane-1,4-diyle ou 1,4-phénylène ; L⁶ et L⁷ représentent chacun, indépendamment, un atome d'hydrogène ou de fluor, sous réserve que L⁶ et L⁷ ne représentent pas simultanément un atome d'hydrogène ; Z⁶ et Z⁷ représentent chacun, indépendamment, un groupe -(CH₂)₂-, -COO- ou une liaison simple ; et chaque atome présent dans le composé est substitué par son isotope.

8. Composition de cristal liquide comprenant au moins un composé du type cristal liquide répondant à la définition suivant l'une quelconque des revendications 1 et 2 comme premier constituant ; au moins un composé choisi dans le groupe consistant en les composés représentés par les formules générales (9), (10) et (11) définis dans la revendication 6 comme deuxième constituant ; et au moins un composé choisi dans le groupe consistant en les composés représentés par les formules générales (12), (13) et (14) définis dans la revendication 7 comme troisième constituant.

9. Composition de cristal liquide comprenant au moins un composé du type cristal liquide répondant à la définition suivant l'une quelconque des revendications 1 et 2 comme premier constituant ; au moins un composé choisi dans le groupe consistant en les composés représentés par les formules générales (7) et (8) définis dans la revendication 5 comme deuxième constituant ; et au moins un composé choisi dans le groupe consistant en les composés représentés par les formules générales (9), (10) et (11) définis dans la revendication 6 comme troisième constituant.

10. Composition de cristal liquide comprenant au moins un composé du type cristal liquide répondant à la définition suivant l'une quelconque des revendications 1 et 2 comme premier constituant ; au moins un composé choisi dans le groupe consistant en les composés représentés par les formules générales (4), (5) et (6) définis dans la revendication 4 comme deuxième constituant ; au moins un composé choisi dans le groupe consistant en les composés représentés par les formules générales (7) et (8) définis dans la revendication 5 comme troisième constituant ; et au moins un composé choisi dans le groupe consistant en les composés représentés par les formules générales (9), (10) et (11) définis dans la revendication 6 comme quatrième constituant.

11. Composition de cristal liquide comprenant au moins un composé optiquement actif en plus d'une composition de cristal liquide répondant à la définition suivant l'une quelconque des revendications 3 à 10.

12. Dispositif d'affichage à cristaux liquides constitué par l'utilisation d'une composition de cristal liquide répondant à la définition suivant l'une quelconque des revendications 3 à 11.
